(19) Europäisches Patentamt
European Patent Office
Office européen des brevets

(11) Veröffentlichungsnummer: **0 385 204 A1**

(12) **EUROPÄISCHE PATENTANMELDUNG**

(21) Anmeldenummer: **90103080.9**

(51) Int. Cl.⁵: **C09B 62/507, D06P 1/384**

(22) Anmeldetag: **17.02.90**

Geänderte Patentansprüche gemäss Regel 86 (2) EPÜ.

(30) Priorität: **21.02.89 DE 3905270**

(43) Veröffentlichungstag der Anmeldung: **05.09.90 Patentblatt 90/36**

(84) Benannte Vertragsstaaten: **BE CH DE FR GB IT LI**

(71) Anmelder: **HOECHST AKTIENGESELLSCHAFT**
**Postfach 80 03 20**
**D-6230 Frankfurt am Main 80(DE)**

(72) Erfinder: **Hähnle, Reinhard, Dr.**
**Kastanienweg 7a**
**D-6240 Königstein/Taunus(DE)**

(54) **Wasserlösliche faserreaktive Azofarbstoffe, deren Bis(carbonsäureamid)-benzol-Vorprodukte, Verfahren zu ihrer Herstellung und ihre Verwendung.**

(57) Azofarbstoffe entsprechend der allgemeinen Formel D-N=N-K° , in welcher bedeuten:
D ist ein Rest der allgemeinen Formel

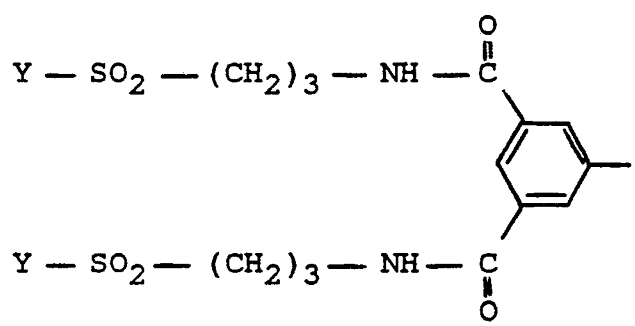

in welcher bedeuten:
Y ist Vinyl, β-Sulfatoethyl, β-Acetyloxyethyl, β-Phosphatoethyl oder β-Thiosulfatoethyl; K° ist der Rest einer Kupplungskomponente der Benzol- oder Naphthalinreihe oder der heterocyclischen Reihe oder Acetoacetarylid-Reihe oder ist ein Rest der allgemeinen Formel -K-G-Q , in welcher K ein bivalenter Rest aus der Benzol- oder Naphthalinreihe ist, G eine Carbonamid- oder Sulfonamid-oder eine Azogruppe und Q einen aliphatischen oder aromatischen Rest, der farbstoffübliche Reste enthält, bedeutet, oder K° ist der Rest einer Kupplungskomponente der Benzol- oder Naphthalinreihe oder der heterocyclischen Reihe oder Acetoacetarylid-Reihe, der eine faserreaktive Acylaminogruppe enthält, wie beispielsweise einen s-Triazinylaminorest, der durch Fluor oder Chlor substituiert sein kann und/oder eine aliphatisch- oder aromatisch substituierte Oxy- oder Aminogruppe besitzt, die eine faserreaktive Gruppierung besitzen kann, des weiteren Vorprodukte entsprechend der allgemeinen Formel

EP 0 385 204 A1

Xerox Copy Centre

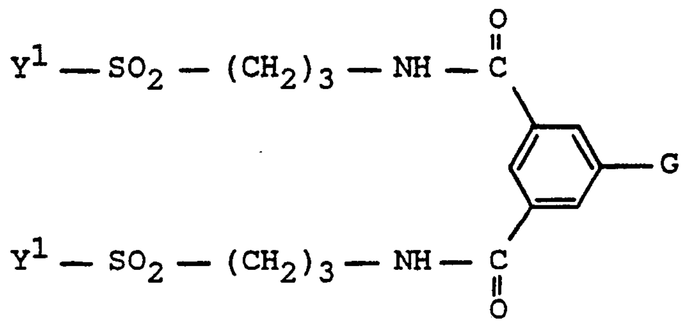

die zur Synthese der Azofarbstoffe dienen können, in welcher G eine Nitrogruppe oder die Aminogruppe und $Y^1$ Vinyl, β-Sulfatoethyl, β-Acetyloxyethyl, β-Thiosulfatoethyl, β-Phosphatoethyl oder β-Hydroxyethyl ist.

Die faserreaktiven Azofarbstoffe färben carbonamidgruppen-und/oder hydroxygruppenhaltiges Material, wie synthetische Polyamidfasern und Wolle und insbesonder Cellulosefasermaterialien, wie Baumwolle, in farbstarken, echten Tönen. Sie zeichnen sich insbesondere durch eine hohe Faser-Farbstoff-Bindungsstabilität und gute Farbausbeute aus.

# Wasserlösliche faserreaktive Azofarbstoffe, deren Bis-(carbonsäureamid)-benzol-Vorprodukte, Verfahren zu ihrer Herstellung und ihre Verwendung

Die Erfindung liegt auf dem technischen Gebiet der faserreaktiven Farbstoffe.

Die Praxis des Färbens mit Reaktivfarbstoffen hat in neuerer Zeit zu erhöhten Anforderungen an die Qualität der Färbungen und die Wirtschaftlichkeit des Färbeprozesses geführt. Infolgedessen besteht weiterhin ein Bedarf an neuen Reaktivfarbstoffen, die verbesserte Eigenschaften, insbesondere in Bezug auf die auf der Faser erzielbare Farbausbeute und die Echtheiten, besitzen. Zwar sind aus der Europäischen Patentanmeldungs-Veröffentlichung Nr. 0 221 013 faserreaktive Farbstoffe mit u.a. einer β-(Vinylsulfonyl)-ethylamido- oder β-(β′-Chlorethylsulfonyl)-ethylamido-Gruppe bekannt, gleichwohl lag der vorliegenden Erfindung die Aufgabe zugrunde, neue, verbesserte Reaktivfarbstoffe zu finden, die Baumwolle in allen Farbtönen färben. Die neuen Farbstoffe sollten sich vor allem durch eine hohe Faser-Farbstoff-Bindungsstabilität auszeichnen und sollten besonders für das Färben nach dem Klotzverfahren geeignet sein; desweiteren sollten sich die nicht auf der Faser fixierten Anteile leicht auswaschen lassen. Desweiteren mußten die mit den neuen Farbstoffen erhältlichen Färbungen gute Allgemeinechtheiten, beispielsweise gute Licht- und Naßechtheiten, besitzen.

Mit der vorliegenden Erfindung wurde diese Aufgabe durch Auffindung der Verbindungen der allgemeinen Formel (1)

$D - N = N - K^o$ (1)

gelöst. In dieser Formel bedeuten:

D ist ein Rest der allgemeinen Formel (2)

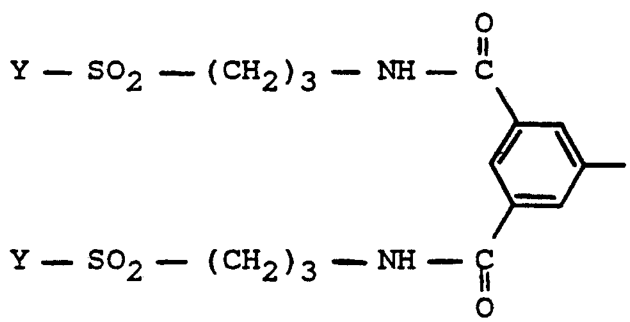

(2)

in welcher

Y die Vinylgruppe oder die β-Sulfatoethyl-, β-Acetyloxyethyl-, β-Thiosulfatoethyl- oder β-Phosphatoethyl-Gruppe, bevorzugt die Vinylgruppe und insbesondere die β-Sulfatoethyl-Gruppe, ist;

M ist ein Wasserstoffatom oder ein Alkalimetall, wie Natrium, Kalium oder Lithium;

$K^o$ ist der Rest einer Kupplungskomponente der Benzol- oder Naphthalinreihe oder der heterocyclischen Reihe oder der Acetoacetarylid-Reihe oder

$K^o$ ist ein Rest der allgemeinen Formel (3)

- K- G- Q (3)

in welcher

K ein bivalenter Rest aus der Benzol- oder Naphthalin-Reihe, wie beispielsweise ein bivalenter Rest einer Kupplungskomponente der Benzol- oder Naphthalinreihe, ist,

G für eine Gruppe der Formel

- N - CO-
  I
  R

oder - N - $SO_2$ -
  I
  R

steht, in welchen

R ein Wasserstoffatom oder eine Alkylgruppe von 1 bis 4 C-Atomen, wie die Methyl- oder Ethylgruppe, ist, oder

G eine Azogruppe der Formel -N=N- ist und

Q einen aliphatischen oder aromatischen Rest, wie einen Alkylenrest von 1 bis 4 C-Atomen oder Phenylenrest, bedeutet, der seinerseits farbstoffübliche Reste, insbesondere faserreaktive Reste, insbesondere solche der allgemeinen Formel $-SO_2-Y$ mit Y der obigen Bedeutung, enthält, oder

$K^o$ ist ein Rest der allgemeinen Formel $-K^*-Z$ ,
in welcher
$K^*$ der bivalente Rest einer Kupplungskomponente der Benzol- oder Naphthalin-Reihe oder der heterocyclischen Reihe oder der Acetoacetarylid-Reihe ist und
Z einen faserreaktiven Acylaminorest bedeutet, wie bevorzugt einen Rest der allgemeinen Formel (4)

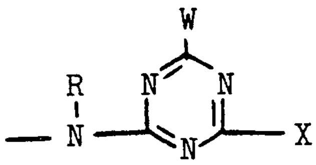 (4)

worin
R eine der obengenannten Bedeutungen hat,
X für ein Fluor- oder Chloratom oder den Morpholinorest oder eine Gruppe der allgemeinen Formel $-O-R^{\alpha}$ oder eine Aminogruppe der allgemeinen Formel $-NR^{\alpha}R^{\beta}$ steht,
in welchen
$R^{\alpha}$ ein Wasserstoffatom oder ein aliphatischer oder aromatischer Rest ist, der farbstoffübliche Reste, einschließlich faserreaktive Reste, bspw. solche der allgemeinen Formel $-SO_2-Y$ mit Y der obigen Bedeutung, enthält, wie bspw. eine Alkylgruppe von 1 bis 4 C-Atomen, wie die Methyl- und Ethylgruppe, ist, die durch Hydroxy, Sulfato, Sulfo, Carboxy, Phosphato, Phenyl, Methoxy oder Ethoxy substitutiert sein kann, oder ein Phenylrest, der durch Substituenten substituiert sein kann, die aus der Gruppe Alkyl von 1 bis 4 C-Atomen, wie Methyl und Ethyl, Alkoxy von 1 bis 4 C-Atomen, wie Methoxy und Ethoxy, Carboxy, Sulfo, einer Gruppe der Formel $-SO_2-Y$ mit Y einer der obengenannten Bedeutungen, Chlor und Brom ausgewählt sind, und
$R^{\beta}$ ein Wasserstoffatom oder eine Alkylgruppe von 1 bis 4 C-Atomen, wie die Methyl- oder Ethylgruppe ist, die durch Hydroxy, Sulfato, Sulfo, Carboxy, Phosphato, Phenyl, Methoxy oder Ethoxy substituiert sein kann, und
W einen der für X angegebenen Bedeutungen besitzt, wobei X und W zueinander gleich oder voneinander verschieden sein können.

Vorzugsweise ist W ein Chlor- oder Fluoratom oder eine Hydroxygruppe.

Die Reste $K^o$ und K entstammen vorzugsweise den Resten der Kupplungskomponenten der Anilin-, Naphthylamin-, Naphthol-, Pyrazolon-, Acetoacetylarylid- und Pyridon-Reihe; sie können faserreaktive Gruppen besitzen.

Kupplungskomponenten der Formel $H-K^o$ der Anilin- und Naphthalinreihe sind beispielsweise die Aniline, N-mono-und N,N-disubstituierten Aniline, m-Phenylendiamine und deren Derivate, Naphtholsulfonsäuren, Aminonaphthaline und deren Sulfonsäurederivate, Naphthole und deren Derivate und Hydroxynaphthoesäurederivate sowie Aminonaphtholsulfonsäuren.

Bevorzugte erfindungsgemäße Azofarbstoffe der allgemeinen Formel (1a) sind beispielsweise Farbstoffe der allgemeinen Formeln (5a), (5b) und (5c)

$D - N = N \{E - N = N\}_v - K^o$ (5a)

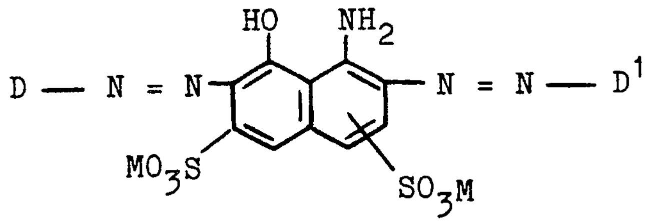

(5b)

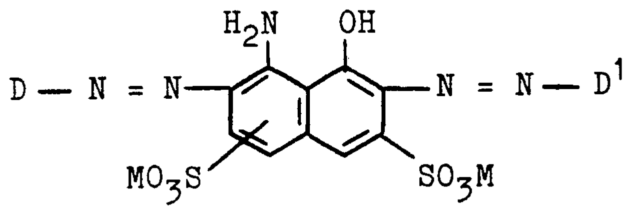

(5c)

in welchen
K°, D und M die obengenannten Bedeutungen haben,
$D^1$ ein Rest einer Diazokomponente der Benzol- oder Naphthalinreihe ist, der durch in faserreaktiven Farbstoffen übliche Substituenten substituiert sein kann, wie beispielsweise durch Substituenten, die aus der folgenden Gruppe vom Substituenten ausgewählt sind: Alkyl von 1 bis 4 C-Atomen, wie Methyl und Ethyl, Alkoxy von 1 bis 4 C-Atomen wie Methoxy und Ethoxy, Carboxy, Sulfo, Halogen, wie Chlor und Brom, Trifluormethyl, Nitro, Benzoylamino, eine Gruppe der oben definierten allgemeinen Formel $-SO_2-Y$, eine faserreaktive aliphatische oder heterocyclische Acylaminogruppe Z, wie beispielsweise eine Gruppe der oben genannten und definierten allgemeinen Formel (4), Alkanoylamino von 2 bis 5 C-Atomen, wie Acetylamino, und Hydroxy; oder
$D^1$ ein Rest der allgemeinen Formel (2) ist,
E den bivalenten Rest einer kupplungsfähigen und diazotierbaren Verbindung bedeutet und
v für die Zahl Null oder 1 steht.

Die Gruppen "Sulfo", "Carboxy", "Thiosulfato", "Phosphato" und "Sulfato" schließen sowohl deren Säureform als auch deren Salzform ein. Demgemäß bedeuten Sulfogruppen Gruppen entsprechend der allgemeinen Formel $-SO_3M$, Carboxygruppen Gruppen entsprechend der allgemeinen Formel -COOM, Thiosulfatogruppen Gruppen entsprechend der allgemeinen Formel $-S-SO_3M$, Phosphatogruppen Gruppen entsprechend der allgemeinen Formel $-OPO_3M_2$ und Sulfatogruppen Gruppen entsprechend der allgemeinen Formel $-OSO_3M$, jeweils mit M der obengenannten Bedeutung.

Die erfindungsgemäßen Verbindungen der allgemeinen Formel (1) können sowohl in saurer Form als auch in Form ihrer Salze, insbesondere der obengenannten Alkali- und Erdalkalimetallsalze, vorliegen. Sie finden, bevorzugt in Form der Alkalimetallsalze, Verwendung zum Färben (einschließlich des Bedruckens) von hydroxygruppen-und/oder carbonamidgruppen-haltigen Materialien, insbesondere Fasermaterialien.

Aromatische Reste $D^1$ der Diazokomponenten $D^1-NH_2$ der Anilin-und Aminonaphthalinreihe sind beispielsweise Reste der allgemeinen Formeln (6a) und (6b)

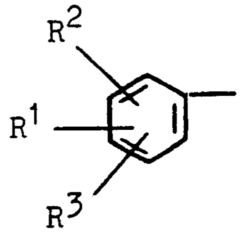

(6a)

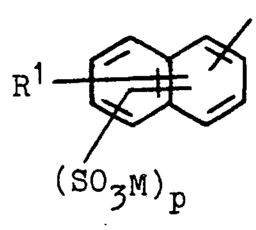

(6b)

in welchen
$R^1$ ein Wasserstoffatom, eine Sulfogruppe oder eine Gruppe der allgemeinen Formel $-SO_2-Y$ mit Y der obengenannten Bedeutung ist,
$R^2$ Wasserstoff, Methyl, Ethyl, Methoxy, Ethoxy, Alkanoyl von 2 bis 5 C-Atomen, wie Acetyl und Propionyl, Cyano, Carboxy, Sulfo, Alkoxycarbonyl von 2 bis 5 C-Atomen, Carbamoyl, N-($C_1$-$C_4$-Alkyl)-carbamoyl,

Fluor, Chlor, Brom oder Trifluormethyl ist,
$R^3$ Wasserstoff, Methyl, Ethyl, Methoxy, Ethoxy, Cyan, Carboxy, Sulfo, Alkanoylamino von 2 bis 5 C-Atomen, wie Acetylamino, Alkoxycarbonyl von 2 bis 5 C-Atomen, Carbamoyl, N-($C_1$-$C_4$-Alkyl)-carbamoyl, Fluor, Chlor, Nitro, Sulfamoyl, N-($C_1$-$C_4$-Alkyl)-sulfamoyl, Alkylsulfonyl von 1 bis 4 C-Atomen, Phenylsulfonyl oder Phenoxy ist,
p die Zahl Null, 1 oder 2 bedeutet (wobei diese Gruppe im Falle von p gleich Null ein Wasserstoffatom bedeutet) und
M die obengenannte Bedeutung hat.

Bevorzugt ist hiervon $R^2$ gleich Wasserstoff, Methyl, Methoxy, Brom, Chlor, Carboxy und Sulfo sowie $R^3$ gleich Wasserstoff, Methyl, Methoxy, Chlor, Carboxy, Sulfo und Acetylamino.

Aromatische Amine der allgemeinen Formel $D^1$-$NH_2$ entsprechend den Formeln (6a) und (6b) sind beispielsweise: 2-Amino- oder 4-Aminobenzoesäure, 3-Amino-benzoesäure, 3-Chloranilin-6-carbonsäure, Anilin-2- oder -3- oder -4-sulfonsäure, 2,5-Disulfo-anilin, 2,4-Disulfo-anilin, 3,5-Disulfoanilin, 2-Aminotoluol-4-sulfonsäure, 2-Amino-anisol-4-sulfonsäure, 2-Amino-anisol-5-sulfonsäure, 4-Amino-anisol-2-sulfonsäure, 2-Ethoxy-anilin-5-sulfonsäure, 2-Ethoxy-anilin-4-sulfonsäure, 4-Sulfo-2-aminobenzoesäure, 2,5-Dimethoxy-anilin-4-sulfonsäure, 2,4-Dimethoxyanilin-5-sulfonsäure, 2-Methoxy-5-methyl-anilin-4-sulfonsäure, 4-Amino-anisol-3-sulfonsäure, 4-Amino-toluol-3-sulfonsäure, 2-Amino-toluol-5-sulfonsäure, 2-Chlor-anilin-4-sulfonsäure, 2-Chlor-anilin-5-sulfonsäure, 2-Brom-anilin-4-sulfonsäure, 2,6-Dichloranilin-4-sulfonsäure, 2,6-Dimethylanilin-3-sulfonsäure oder -4-sulfonsäure, 3-Acetylamino-6-sulfoanilin, 4-Acetyl-amino-2-sulfo-anilin, 1-Aminonaphthalin-4-sulfonsäure, 1-Aminonaphthalin-3-sulfonsäure, 1-Aminonaphthalin-5-sulfonsäure, 1-Aminonaphthalin-6-sulfonsäure, 1-Aminonaphthalin-7-sulfonsäure, 1-Aminonaphthalin-3,7-disulfonsäure, 1-Aminonaphthalin-3,6,8-trisulfonsäure, 1-Aminonaphthalin-4,6,8-trisulfonsäure, 2-Naphthylamin-5-sulfonsäure oder -6- oder -8-sulfonsäure, 2-Aminonaphthalin-3,6,8-trisulfonsäure, 2-Aminonaphthalin-6,8-disulfonsäure, 2-Aminonaphthalin-1,6-disulfonsäure, 2-Aminonaphthalin-1-sulfonsäure, 2-Aminonaphthalin-1,5-disulfonsäure, 2-Aminonaphthalin-3,6-disulfonsäure, 2-Aminonaphthalin-4,8-disulfonsäure, 4-(β-Sulfatoethylsulfonyl)-anilin, 3-(β-Sulfatoethylsulfonyl)-anilin, 2-Sulfo-5-(β-sulfatoethylsulfonyl)-anilin, 2-Sulfo-4-(β-sulfatoethylsulfonyl)-anilin, 2-Methoxy-4-(β-sulfatoethylsulfonyl)-anilin, 2-Methoxy-5-methyl-4-(β-sulfatoethylsulfonyl)-anilin, 2,5-Dimethoxy-4-(β-sulfatoethylsulfonyl)-anilin, 2-Amino-5-(β-sulfatoethylsulfonyl)-phenol, 2-Amino-4-(β-sulfatoethylsulfonyl)-phenol, 2-Amino-6-(β-sulfatoethylsulfonyl)-naphthalin-8-sulfonsäure, 2-Amino-8-(β-sulfatoethylsulfonyl)-naphthalin-6-sulfonsäure, 2-Amino-5-(β-sulfatoethylsulfonyl)-naphthalin-7-sulfonsäure und 2-Amino7-(β-sulfatoethylsulfonyl)-naphthalin-5-sulfonsäure sowie 3,5-Bis-|γ-(β′-sulfatoethylsulfonyl)-propylamidocarbonyl]-anilin.

Aromatische Amine der Diazokomponente $D^1$-$NH_2$, die einen faserreaktiven Acylaminorest Z enthalten, wie beispielsweise den Rest der Formel (4), sind bspw. solche der allgemeinen Formel (7a), die sich von den Diaminen der Formel (7b) ableiten:

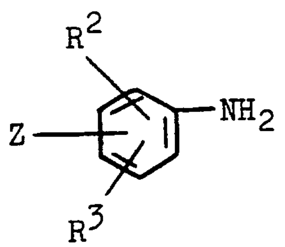
(7a)

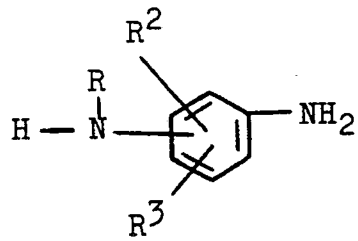
(7b)

in welchen Z, R, $R^2$ und $R^3$ die oben angegebenen, insbesondere bevorzugten Bedeutungen haben.

Amine der allgemeinen Formel (7b) sind beispielsweise: 1,3-Diaminobenzol, 1,3-Diaminobenzol-4-sulfonsäure, 1,3-Diaminobenzol-4,6-disulfonsäure, 1,4-Diaminobenzol, 1,4-Diaminobenzol-2-sulfonsäure, 1,4-Diaminobenzol-2,5-disulfonsäure, 1,4-Diamino-2-methyl-benzol, 1,4-Diamino-2-methoxybenzol, 1,3-Diamino-4-methyl-benzol, 1,5-Diamino-4-methylbenzol-2-sulfonsäure, 1,5-Diamino-4-methoxybenzol-2-sulfonsäure, 1,3-Diaminobenzol-5-sulfonsäure und 1,3-Diamino-5-methylbenzol.

Reste K°, K und K* sind bspw. solche der allgemeinen Formeln (8a) bis (8f)

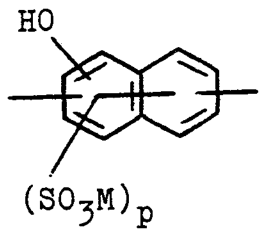
(8a)

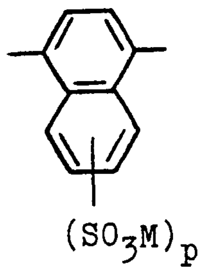
(8b)

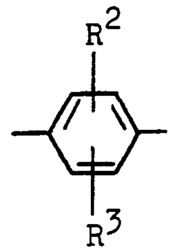
(8c)

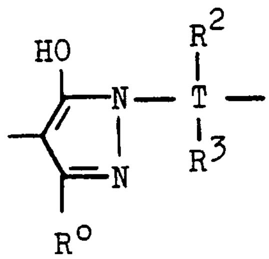
(8d)

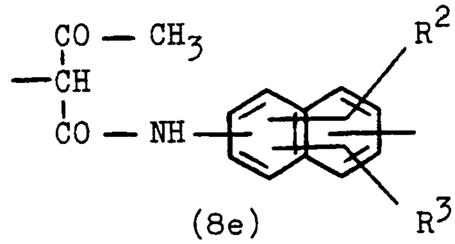
(8e)

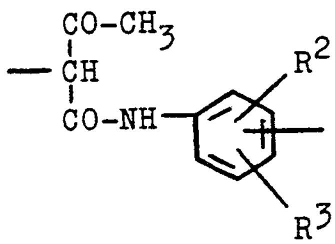
(8f)

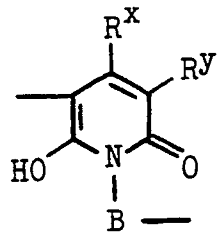
(8g)

in welchen
$R^2$, $R^3$, p und M die obengenannten Bedeutungen haben,
T ein Benzol- oder Naphthalinring ist,
B Alkylen von 1 bis 4 C-Atomen oder Benzyl oder Phenethyl (wobei die freie Bindung am Benzolkern steht) oder Phenylen ist, deren Benzolkerne durch Fluor, Chlor, Brom, Methyl, Methoxy, Cyano, Sulfo, Carboxy, Acetyl, Nitro, Carbamoyl und/oder Sulfamoyl substituiert sein können,
$R^x$ ein Wasserstoffatom oder eine Alkylgruppe von 1 bis 4 C-Atomen, wie die Methylgruppe, oder eine durch Alkoxy von 1 bis 4 C-Atomen, wie Methoxy, oder Cyano substituierte Alkylgruppe von 1 bis 4 C-Atomen ist, bevorzugt Methyl oder Phenyl ist,
$R^y$ ein Wasserstoffatom, eine Sulfogruppe oder eine Sulfoalkylgruppe mit einem Alkylenrest von 1 bis 4 C-Atomen, wie die Sulfomethylgruppe, oder eine Cyano-, Methylsulfonyl-, Phenylsulfonyl- oder Carbamoylgruppe ist und
$R^o$ für Wasserstoff, Alkyl von 1 bis 4 C-Atomen, wie Methyl, Cyano, Carboxy, Carbalkoxy von 2 bis 5 C-Atomen, Carbonamido oder Phenyl steht, bevorzugt Methyl, Carboxy, Carbomethoxy oder Carbethoxy ist.

Aromatische Reste E einer kupplungsfähigen und diazotierbaren Verbindung der Anilin- und Aminonaphthalinreihe leiten sich beispielsweise von Aminen der allgemeinen Formeln (9a) und (9b)

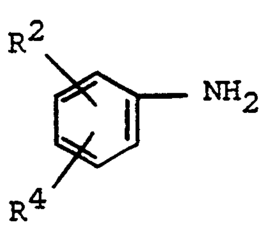
(9a)

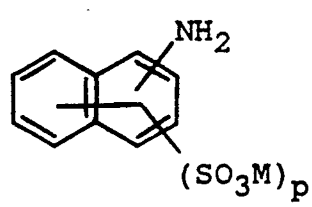
(9b)

ab, in welchen
$R^2$ und M die oben angegebenen Bedeutungen haben,
$R^4$ ein Wasserstoffatom, eine Alkylgruppe von 1 bis 4 C-Atomen, wie die Methyl- oder Ethylgruppe, eine Alkoxygruppe von 1 bis 4 C-Atomen, wie die Methoxy- und Ethoxygruppe, ein Chloratom, eine Alkanoylaminogruppe von 2 bis 5 C-Atomen, wie die Acetylamino- und Propionylaminogruppe, die Benzoylaminogruppe, die Ureidogruppe, eine Phenylureidogruppe, eine Alkylureidogruppe mit 1 bis 4 C-Atomen im Alkylrest, eine Phenylsulfonylgruppe oder eine Alkylsulfonylgruppe von 1 bis 4 C-Atomen ist und
p für die Zahl Null, 1 oder 2 steht (wobei diese Gruppe im Falle von p gleich Null ein Wasserstoffatom bedeutet).

Solche Verbindungen sind beispielsweise:
Anilin, 3-Methylanilin, 3-Chloranilin, 2,5-Dimethylanilin, 2,5-Dimethoxyanilin, 3-Methoxyanilin, 3-Methyl-6-methoxyanilin, 3-Aminophenyl-harnstoff, 3-Acetylamino-6-methylanilin, 2-Amino-4-acetyl-aminobenzol-1-sulfonsäure, 1-Aminonaphthalin, 1-Aminonaphthalin-6- oder -7- oder -8-sulfonsäure, 3-Acetylaminoanilin, 2-Methylanilin, 2-Methoxyanilin, 3-Benzoylamino-anilin, 2,3-Dimethylanilin, 3,5-Dimethylanilin, 1-Amino-2-methoxy-5-acetylamino-benzol.

Kupplungskomponenten der Formel H-K° sind beispielsweise Verbindungen der allgemeinen Formeln (10a) bis (10g)

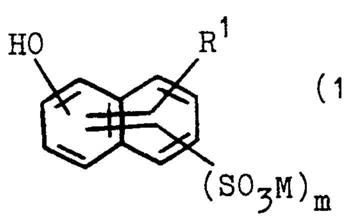
(10a)

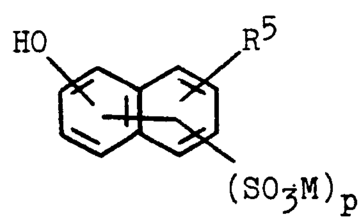
(10b)

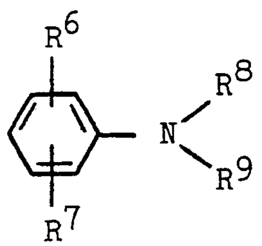
(10c)

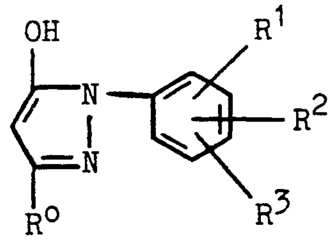
(10d)

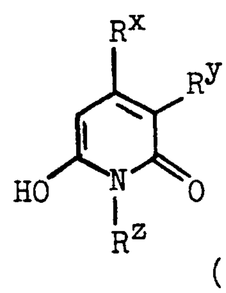
(10e)

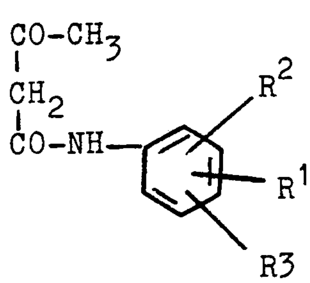
(10f)

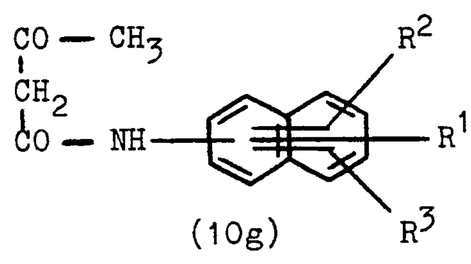
(10g)

in welchen
$R^1$, $R^2$, $R^3$, $R^o$, $R^x$, $R^y$, p und M die obengenannten Bedeutungen haben,
m für die Zahl Null, 1, 2 oder 3 steht (wobei diese Gruppe im Falle m gleich Null ein Wasserstoffatom bedeutet),
$R^5$ Alkylureido mit Alkylgruppen von 1 bis 6 C-Atomen, Phenylureido, im Phenylrest durch Chlor, Methyl, Methoxy, Nitro, Sulfo und/oder Carboxy substituiertes Phenylureido, Alkanoylamino von 2 bis 7 C-Atomen, wie beispielsweise Acetylamino und Propionylamino, Cyclohexanoylamino, Benzoylamino oder im Benzolrest durch Chlor, Methyl, Methoxy, Nitro, Sulfo und/oder Carboxy substituiertes Benzoylamino bedeutet,
$R^6$ Wasserstoff, Alkyl von 1 bis 4 C-Atomen, wie Methyl und Ethyl, Alkoxy von 1 bis 4 C-Atomen, wie

Methoxy und Ethoxy, Brom, Chlor oder Alkanoylamino von 2 bis 7 C-Atomen, wie Acetylamino und Propionylamino, ist,
$R^7$ Wasserstoff, Alkyl von 1 bis 4 C-Atomen, wie Methyl und Ethyl, Alkoxy von 1 bis 4 C-Atomen, wie Methoxy und Ethoxy, Chlor oder Alkanoylamino von 2 bis 7 C-Atomen, wie Acetylamino und Propionylamino, eine Ureido- oder Phenylureidogruppe ist,
$R^8$ Wasserstoff oder Alkyl von 1 bis 4 C-Atomen, das durch Hydroxy, Cyan, Carboxy, Sulfo, Sulfato, Methoxycarbonyl, Ethoxycarbonyl oder Acetoxy substituiert sein kann, ist,
$R^9$ für Alkyl von 1 bis 4 C-Atomen, das durch Hydroxy, Cyan, Carboxy, Sulfo, Sulfato, Methoxycarbonyl, Ethoxycarbonyl oder Acetoxy substituiert sein kann, steht oder Benzyl oder Phenyl oder durch Alkyl von 1 bis 4 C-Atomen, Alkoxy von 1 bis 4 C-Atomen, Chlor und/oder Sulfo substituiertes Phenyl ist und
$R^z$ ein Wasserstoffatom oder eine Alkylgruppe von 1 bis 6 C-Atomen, bevorzugt von 1 bis 4 C-Atomen ist, die durch Phenyl, Sulfo, Sulfato, Carboxy, Sulfophenyl, Hydroxy, Amino, Methoxy, Ethoxy, Acetylamino, Benzoylamino oder Cyano substituiert sein kann, oder ist Cyclohexyl, Phenyl oder durch Carboxy, Sulfo, Benzoylamino, Acetylamino, Methyl, Methoxy, Cyano und/oder Chlor substituiertes Phenyl.

Verbindungen der allgemeinen Formeln (10) sind beispielsweise: 1-Naphthol3-sulfonsäure, 1-Naphthol-4-sulfonsäure, 1-Naphthol-5-sulfonsäure, 1-Naphthol-8-sulfonsäure, 1-Naphthol-3,6-disulfonsäure, 1-Naphthol-3,8-disulfonsäure, 2-Naphthol-5-sulfonsäure, 2-Naphthol-6-sulfonsäure, 2-Naphthol-7-sulfonsäure, 2-Naphthol-8-sulfonsäure, 2-Naphthol-3,6-disulfonsäure, 2-Naphthol-6,8-disulfonsäure, 2-Naphthol-3,6,8-trisulfonsäure, 1-Acetylamino-8-hydroxynaphthalin-3,6-disulfonsäure, 1-Benzoylamino-8-hydroxynaphthalin-3,6-disulfonsäure, 1-Acetylamino-8-hydroxynaphthalin-4,6-disulfonsäure, 1-Benzoylamino-8-hydro xynaphthalin-4,6-disulfonsäure, 2-Acetylamino-5-hydroxy-naphthalin-7-sulfonsäure, 3-Acetylamino-5-hydroxy-naphthalin-7-sulfonsäure, 2-Methyl-amino-8-hydroxy-naphthalin-6-sulfonsäure oder 2-(3′- und 4′-Sulfophenyl)-amino-8-hydroxynaphthalin-6-sulfonsäure, 3-(3′- und 4′-Sulfophenyl)-amino-8-hydroxy-naphthalin-6-sulfonsäure, N,N-Di-(β-sulfoethyl)-anilin und dessen im Benzolkern durch Methyl, Methoxy und/oder Ethoxy mono- oder disubstituierten Derivate, N-Ethyl-N-(β-sulfoethyl)-anilin, N-(β-Sulfoethyl)anilin, N-(β-Carboxyethyl)-anilin und deren im Benzolkern durch Methyl, Methoxy und/oder Ethoxy mono- oder disubstituierten Derivate, sowie 1-[4′-(β-Sulfatoethyl-sulfonyl)-2′-sulfo]-phenyl-3-methyl-pyrazol-5-on, 1-[4′-(β-Sulfatoethylsulfonyl)]-phenyl-3-carboxy-pyrazol-5-on und 1-(4′-Sulfophenyl)-3-carboxy-pyrazol-5-on.

Von besonderer Bedeutung sind sulfogruppenhaltige, gegebenenfalls Azogruppen, wie 1 oder 2 Azogruppen, tragende Kupplungskomponenten, die in o- oder p-Stellung zu einer Hydroxy- und/oder Aminogruppe kuppeln, wie beispielsweise 2-Acetylamino-5-hydroxy-naphthalin-7-sulfonsäure, 2-Acetyl-amino-8-hydroxynaphthalin-6- sulfonsäure, 1-Acetylamino-8-hydroxynaphthalin-3,6-disulfonsäure, 1-Benzoylamino-8-hydroxynaphthalin-3,6-disulfonsäure, 1-Acetylamino-8-hydroxynaphthalin-4,6-disulfonsäure oder 1-Benzoylamino-8-hydroxynaphthalin-4,6-disulfonsäure.

Als Kupplungskomponenten H-K° sind weiterhin besonders zu nennen: 1-Amino-8-hydroxynaphthalin-3,6- und -4,6-disulfonsäure sowie deren durch saure Kupplung erhaltene Arylazokupplungsprodukte der allgemeinen Formel (11)

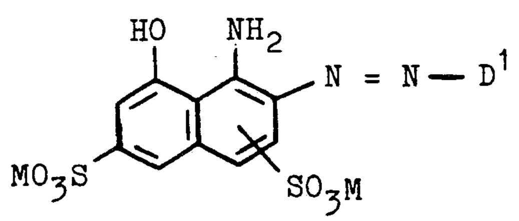

(11)

in welcher $D^1$ und M die obengenannten Bedeutungen haben.

Einzelne Reste $D^1$ sind beispielsweise: Phenyl, 2-Sulfo-phenyl, 3-Sulfo-phenyl, 4-Sulfo-phenyl, 2,4-Disulfo-phenyl, 2,5-Disulfo-phenyl, 3,5-Disulfo-phenyl, 1,5-Disulfo-naphth-2-yl, 4,8-Disulfo-naphth-2-yl, 3,6,8-Trisulfo-naphth-2-yl, 4,6,8-Trisulfo-naphth-2-yl, 3,6,8-Trisulfo-naphth-1-yl, 4,6,8-Trisulfo-naphth-1-yl, 4-Sulfo-naphth-1-yl, 1-Sulfo-naphth-2-yl, 3-Acetylamino-phenyl, 4-Acetylamino-phenyl, 4-Acetylamino-2-sulfo-phenyl, 5-Acetylamino-2-sulfo-phenyl, 4-Nitro-phenyl, 4-Nitro-2-sulfo-phenyl, 6-Acetylamino-4,8-disulfo-naphth-2-yl, 4-(β-Sulfatoethylsulfonyl)-phenyl, 3-(β-Sulfatoethylsulfonyl)-phenyl, 2-Sulfo-4-(β-sulfatoethylsulfonyl)-phenyl, 2-Methoxy-5-(β-sulfatoethylsulfonyl)-phenyl, 2,5-Dimethoxy-4-(β-sulfatoethylsulfonyl)-phenyl, 1-Sulfo-6-(β-sulfatoethylsulfonyl)-naphth-2-yl.

Pyrazolon-Kupplungskomponenten sind beispielsweise 3-Methyl-, 3-Carboxy- und 3-($C_2$-$C_5$-Alkoxycarbonyl)-5-pyrazolone, die in 1-Stellung Wasserstoff, gegebenenfalls durch Methyl, Ethyl, Fluor, Chlor, Brom, Trifluormethyl, Methoxy, Ethoxy, Cyano, Phenoxy, Phenylsulfonyl, Methylsulfonyl, Sulfo, Benzoyl, Acetyl,

Acetylamino, Nitro, Hydroxy, Carboxy, Carbamoyl oder Sulfamoyl substituiertes Phenyl oder sulfosubstituiertes 1- oder 2-Naphthyl tragen, beispielsweise:
1-(2′-Methoxy-5′-methylphenyl)-, 1-(2′-Chlor-5′-sulfophenyl)-, 1-(2′-Methoxy-5′-sulfophenyl)-, 1-(2′-Methyl-4′-sulfophenyl)-, 1-(2′,5′-Dichlor-4′-sulfophenyl)-, 1-(2′,5′-Disulfophenyl)-, 1-(2′-Carboxyphenyl)-, 1-(3′-Sulfophenyl)-, 1-(4′-Sulfophenyl)-, 1-(3′-Sulfamoylphenyl)-3-carboxy-5-pyrazolon, 1-(3′- oder 4′-Sulfophenyl)-, 1-(2′-Chlor-4′- oder -5′-sulfophenyl)-, 1-(2′-Methyl-4′-sulfophenyl)-, 1-(4′,8′-Disulfo-8-naphthyl)-, 1-(6′-Sulfo-1-naphthyl)-3-methyl-5-pyrazolon, 1-Phenyl-5-pyrazolon-3-carbonsäureethylester, 5-Pyrazolon-3-carbonsäureethylester oder 5-Pyrazolon-3-carbonsäure.

Pyridonkupplungskomponenten sind beispielsweise:
1-Ethyl-2-hydroxy-4-methylscarbonamido-pyridon-6, 1-(2′-Hydroxyethyl)-2-hydroxy-4-methyl-5-carbonamido-pyridon-6, (4′-Sulfo-1-phenyl)-2-hydroxy-4-methyl-5-carbonamido-pyridon-6, 1-(2′-Sulfoethyl)-2-hydroxy-4-methyl-5-cyano-pyridon-6, 1-Ethyl-2-hydroxy-4-sulfomethyl-5-carbonamido-pyridon-6, 1-Ethyl-2-hydroxy-4-methyl-5-sulfomethyl-pyridon-6, 1-Methyl-2-hydroxy-4-methyl-5-cyano-pyridon-6, 1-Methyl-2-hydroxy-5-acetyl-pyridon-6, 1,4-Dimethyl-2-hydroxy-5-cyano-pyridon-6, 1,4-Dimethyl-2-hydroxy-5-carbonamido-pyridon-6, 2,6-Dihydroxy-4-ethyl-5-cyano-pyridin, 2,6-Dihydroxy-4-ethyl-5-carbonamido-pyridin, 1-Ethyl-2-hydroxy-4-methyl-5-sulfomethyl-pyridon-6, 1-Methyl-2-hydroxy-4-methyl-5-methylsulfonyl-pyridon-6, 1-Carboxymethyl-2-hydroxy-4-ethyl-5-phenylsulfonyl-pyridon-6 und 1-(2′-Sulfo-ethyl)-2-hydroxy-4-carboxy-pyridon-6.

Weitere Kupplungskomponenten H-K° mit einer faserreaktiven Gruppe Z sind bspw. solche der allgemeinen Formeln (12a) bis (12e)

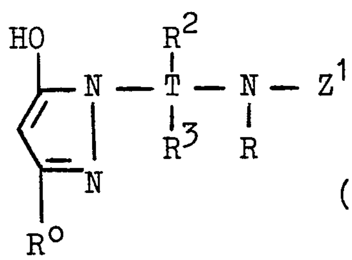

(12a)

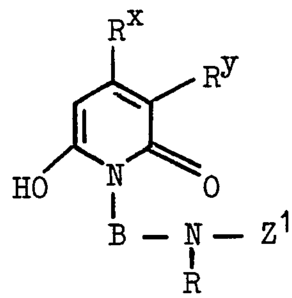

(12b)

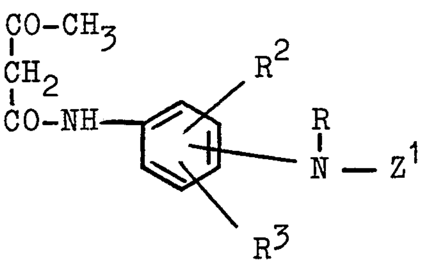

(12c)

oder

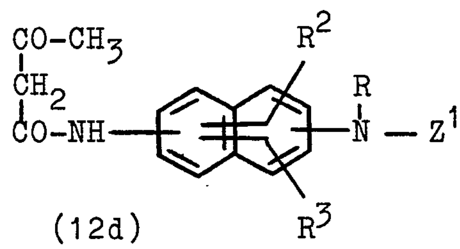

(12d)

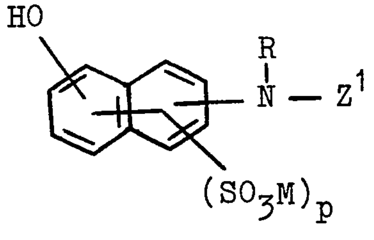

(12e)

in welchen
R, $R^x$, $R^y$, $R^o$, T, $R^2$ und $R^3$ die obengenannten Bedeutungen haben,
$Z^1$ für einen faserreaktiven Acylrest steht, wie beispielsweise für einen Rest der allgemeinen Formel (4a)

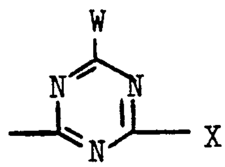

(4a)

mit W und X der obengenannten Bedeutung.

In aminogruppenhaltige Kupplungskomponenten der Formel H-K*-NRH , wie solche, die den Verbindungen der allgemeinen Formeln (12a) bis (12e) entsprechen, die hier jedoch anstelle des Restes $Z^1$ ein Wasserstoffatom besitzen, und solche der allgemeinen Formeln (13a) und (13b)

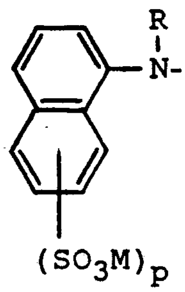

(13a)

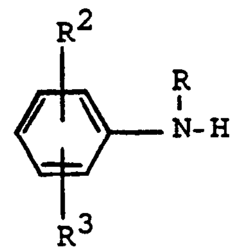

(13b)

mit R, $R^2$, $R^3$, M und p der obengenannten Bedeutungen, die zum Aufbau der erfindungsgemäßen Azoverbindungen (1) dienen, kann durch Umsetzung mit einem faserreaktiven Acylierungsmittel, im Falle der Verbindungen der Formeln (13a) und (13b) nach deren Kupplung, ein faserreaktiver Rest $Z^1$ eingeführt werden.

Prinzipiell kommen hierfür alle bekannten faserreaktiven Acylierungsmittel in Betracht, insbesondere solche der Halogentriazin-Reihe, wie bspw. ein Rest der Formel (14)

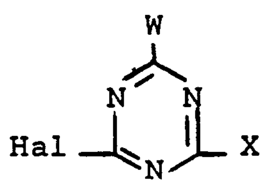

(14)

in welcher
Hal ein Halogenatom, wie Chlor- oder Fluoratom, ist und
W und X die obengenannte Bedeutung haben.

Kupplungskomponenten, in deren freie Aminogruppe bspw. nach beendeter Kupplung der Arylrest $Z^1$ eingeführt werden kann, sind beispielsweise:
1-(3′- oder 4′-Aminophenyl)-, 1-(2′-Sulfo-5′-aminophenyl)-und 1-(2′-Methoxy-5′-aminophenyl)-3-carboxy-5-pyrazolon, 1-(3′- oder 4′-Aminophenyl)-3-methyl-5-pyrazolon und 1-(6′-Amino-4′,8′-disulfonaphthyl-2′)-3-carboxy-5-pyrazolon.

Die vorliegende Erfindung betrifft weiterhin Verfahren zur Herstellung der erfindungsgemäßen Azoverbindungen der allgemeinen Formel (1). Die Verbindungen (1) können beispielsweise erfindungsgemäß hergestellt werden, indem man ein Diazoniumsalz eines Amins der allgemeinen Formel (15)

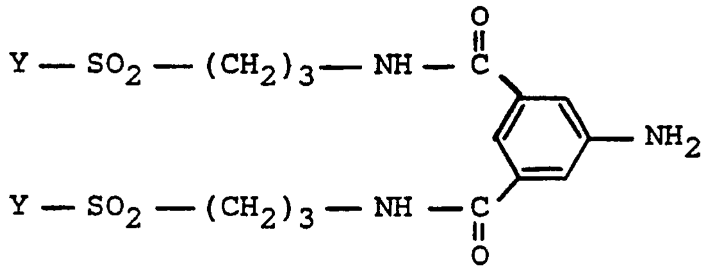

(15)

in welcher Y die obengenannten Bedeutungen haben, mit einer Kupplungskomponente der allgemeinen Formel H-K° mit K° der obengenannten Bedeutung kuppelt.

Erfindungsgemäße Verbindungen (5c) können in erfindungsgemäßer Weise ebenfalls durch Kupplungsreaktion hergestellt werden, indem man ein Diazoniumsalz eines Amins der allgemeinen Formel $D^1-NH_2$ mit $D^1$ der obengenannten Bedeutung mit einer Verbindung der allgemeinen Formel (16)

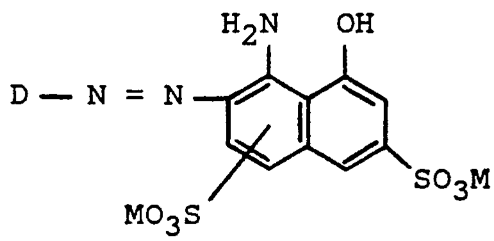

(16)

in welcher D und M die obengenannten Bedeutungen haben, kuppelt.

Die Diazotierungs- und Kupplungsreaktionen erfolgen in üblicher Weise, so die Diazotierung in der Regel bei einer Temperatur zwischen -5° C und +15° C und einem pH-Wert unterhalb von 2 mittels einer starken Säure und Alkalinitrit in bevorzugt wäßrigem Medium und die Kupplungsreaktion in der Regel bei einem pH-Wert zwischen 1 und 4,5 im Falle der aminogruppenhaltigen Kupplungskomponente und bei einem pH-Wert zwischen 3 und 7,5 im Falle der hydroxygruppenhaltigen Kupplungskomponente und bei einer Temperatur zwischen 0 und 25° C, ebenso bevorzugt in wäßrigem Medium.

Die neuen Azoverbindungen der allgemeinen Formel (1), in welcher K° oder damit verbundene Gruppen eine faserreaktive Gruppe Z besitzen, können erfindungsgemäß auch in der Weise hergestellt werden, daß man eine Verbindung der allgemeinen Formel (17)

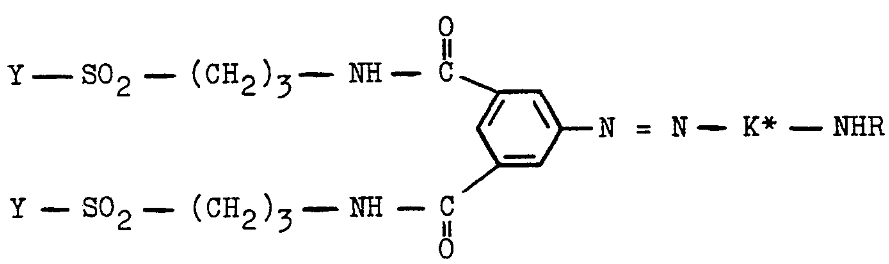

(17)

mit Y, K* und R der obengenannten Bedeutung, wie bspw. auch eine Verbindung der allgemeinen Formel (18)

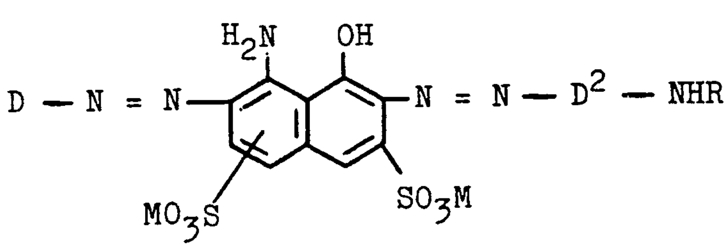

(18)

in welcher D, R und M die obengenannten Bedeutungen haben und
$D^2$ ein Phenylen- oder Naphthylenrest ist, der durch in faserreaktiven Farbstoffen übliche Substituenten substituiert sein kann, wie beispielsweise durch Substituenten aus der Gruppe Alkyl von 1 bis 4 C-Atomen, wie Methyl und Ethyl, Alkoxy von 1 bis 4 C-Atomen wie Methoxy und Ethoxy, Carboxy, Sulfo, Halogen, wie Chlor und Brom, Trifluormethyl, Nitro, Benzoylamino, eine Gruppe der oben definierten allgemeinen Formel $-SO_2-Y$ , Alkanoylamino von 2 bis 5 C-Atomen, wie Acetylamino, und Hydroxy,
mit einer Verbindung der allgemeinen Formel $Hal-Z^1$ mit Hal und $Z^1$ der obengenannten Bedeutung umsetzt.

Erfindungsgemäße Farbstoffe entsprechend der allgemeinen Formel (1) mit der oben angegebenen Bedeutung, in welcher jedoch die beiden Y β-Sulfatoethyl-Gruppen bedeuten und/oder einer der Substituenten von $K^o$ einen β-Sulfatoethyl-Rest enthält, können erfindungsgemäß auch hergestellt werden, indem man eine Ausgangsverbindung entsprechend der allgemeinen Formel (1) mit der für Formel (1) angegebenen Bedeutung, jedoch mit dem Unterschied, daß die Formelreste Y β-Hydroxyethyl-Gruppen darstellen und/oder einer der Substituenten in $K^o$ einen β-Hydroxyethyl-Rest enthält, analog bekannten Verfahrensweisen mit einem Sulfatierungsmittel, wie konzentrierte Schwefelsäure oder Schwefeltrioxyd enthaltende Schwefelsäure, umsetzt, wobei die Sulfatierung in der Regel bei einer Temperatur zwischen 10 und 25 °C erfolgt.

Die Ausgangsverbindungen entsprechend der allgemeinen Formel (1), jedoch mit den β-Hydroxyethylsulfonyl-Gruppen, sind analog den obigen Angaben zur Herstellung der erfindungsgemäßen Farbstoffe der Formel (1) synthetisierbar, indem man von den entsprechenden Ausgangsverbindungen, die diese β-hydroxyethylsulfonyl-Gruppe(n) enthalten, ausgeht.

Faserreaktive Diazokomponentenreste $D^1$ sind beispielsweise 4-(2-m-Sulfophenylamino-4-fluor-s-triazin-6-yl)-amino-2- sulfo-phenyl, 4-(β-Sulfatoethylsulfonyl)-phenyl, 3-(β-Sulfatoethylsulfonyl)-phenyl und 4-Vinylsulfonyl-phenyl.

Die Umsetzung der Halogentriazinylamino-Verbindungen der allgemeinen Formel $Hal-Z^1$ , wie der der allgemeinen Formel (15), mit einer Aminoverbindung der allgemeinen Formel (17) bzw. (18) erfolgt beispielsweise bei einer Temperatur zwischen -10 °C und 60 °C, bevorzugt zwischen 0 und 5 °C, und bei einem pH-Wert zwischen 3 und 10,5, bevorzugt zwischen 4 und 8, wobei darauf zu achten ist, daß die faserreaktive Gruppierungen im schwach alkalischen Bereich nicht geschädigt werden.

Die vorliegende Erfindung betrifft weiterhin Verbindungen entsprechend der allgemeinen Formel (19)

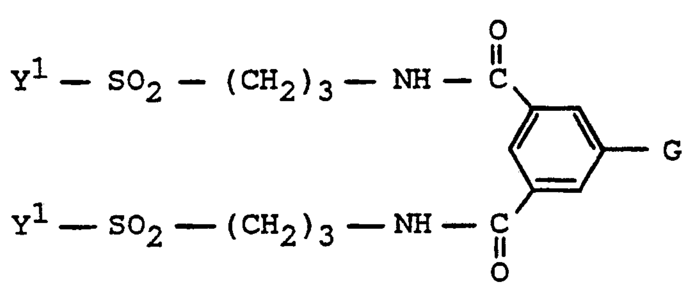

(19)

in welcher $Y^1$ eine der für Y genannten Bedeutungen besitzt oder eine β-Hydroxyethyl-Gruppe bedeutet und G für die Nitrogruppe oder die Aminogruppe steht. Diese erfindungsgemaßen Verbindungen dienen als Ausgangsprodukte zur Herstellung von faserreaktiven Farbstoffen. Hierbei kann die Anilinverbindung gemäß Formel (19), erhältlich durch Reduktion der Nitrogruppe in der Nitrobenzol-Verbindung gemäß Formel (19), direkt als Diazokomponente zur Synthese von faserreaktiven Azofarbstoffen dienen; sie kann aber auch als Aminoverbindung selbst durch Reaktion mit einer für Aminogruppen reaktiven Gruppierung, die in einem Farbstoffrest enthalten ist, in einen bereits bestehenden Farbstoffrest eingeführt werden, wie beispielsweise durch Umsetzung einer Säurechloridgruppe oder einem halogensubstituierten heterocyclischen Rest, wie einem Halogentriazin-Rest eines Farbstoffrestes, analog üblichen Verfahrensweisen.

Die neuen Verbindungen entsprechend der allgemeinen Formel (19) lassen sich in erfindungsgemäßer Weise herstellen, indem man das γ-(β′-Hydroxyethylsulfonyl)-propylamin in zweifach molarer Menge mit 5-Nitro-benzol-1,3-dicarbonsäurechlorid in wäßrigem Medium bei einem pH-Wert zwischen 7 und 9,5 und bei einer Temperatur von 20 bis 50 °C umsetzt und im Falle der Synthese der entsprechenden Anilinverbindung von Formel (19) die so erhaltene Nitrobenzol-Verbindung entsprechend der allgemeinen Formel (19), in welcher $Y^1$ für die β-Hydroxyethyl-Gruppe steht und G die Nitrogruppe bedeutet, gemäß üblicher Verfahrensweise der Reduktion einer aromatischen Nitrogruppe zur Aminogruppe, wie beispielsweise durch katalytische Reduktion mittels Wasserstoff an einem metallischen Katalysator, wie beispielsweise Raney-Nickel, in beispielsweise wäßrigem Medium, zur Anilinverbindung (19) reduziert.

Die Verbindungen der allgemeinen Formel (19), in welchen $Y^1$ für die β-Hydroxyethyl-Gruppe stehen, können in an und für sich bekannter Verfahrensweise in die faserreaktiven Verbindungen mit $Y^1$ der entsprechenden faserreaktiven Gruppierung einer der anfangs angegebenen Bedeutungen überführt werden, so beispielsweise in deren Esterderivate mit $Y^1$ gleich der Sulfato-, Phosphato- oder Acetyloxy- Gruppe durch Umsetzung mit beispielsweise 100%iger Schwefelsäure oder Schwefeltrioxid enthaltender Schwefelsäure bzw. mit konzentrierter Phosphorsäure oder Polyphosphorsäure bzw. mittels Eisessig.

Die vorliegende Erfindung betrifft demgemäß auch die Verwendung der erfindungsgemäßen Zwischenprodukte der allgemeinen Formel (19) zur Synthese von faserreaktiven Farbstoffen, vorzugsweise Azofarbstoffen.

Die erfindungsgemäßen Farbstoffe eignen sich als faserreaktive Farbstoffe zum Färben und Bedrucken von hydroxygruppenhaltigen Fasern, insbesondere von Baumwolle, ebenso auch für synthetische oder natürliche Polyamidfasern, wie Wolle. Als Färbeverfahren eignen sich die bekannten Färbe- und Druckverfahren für faserreaktive Farbstoffe. Solche Verfahrensweisen sind zahlreich in der Allgemein- und Patentliteratur beschrieben, so beispielsweise in der US-PS 4 775 746.

Insbesondere sind die erfindungsgemäßen Farbstoffe im Ausziehverfahren bei 40 bis 80° C und im Kaltverweilverfahren mit Vorteil anwendbar. Die Applikation ist in einem weiten Temperaturbereich möglich, und die Farbstoffe zeichnen sich durch eine hohe Farbtiefe und einen hohen Fixiergrad aus; deren Färbungen und Drucke besitzen hohe Echtheiten.

Die nachstehenden Beispiele dienen zur Erläuterung der Erfindung. Die darin genannten Teile sind Gewichtsteile, (die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säuren angegeben; im allgemeinen werden sie in Form ihrer Natrium- oder Kaliumsalze hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalisalze, in die Synthese eingesetzt werden.

Die für die erfindungsgemäßen Verbindungen angegebenen Absorptionsmaxima ($\lambda_{max}$) im sichtbaren Bereich wurden anhand derer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

**Beispiel A**

Die Ausgangsverbindung der Formel (19A)

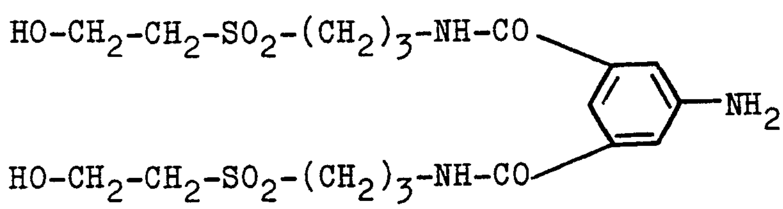

(19A)

wird wie folgt hergestellt:

In 750 Teile 3-Amino-propan-1-ol leitet man bis zur Bildung des Hydrochlorids etwa 365 Teile Chlorwasserstoff ein. In das noch flüssige Hydrochlorid werden bei 70 bis 85° C langsam 1190 Teile Thionylchlorid gegeben. Das nunmehr schwer rührbare Reaktionsgemisch wird noch eine Stunde gerührt und danach in 600 Teilen Wasser gelöst. Die Lösung gibt man langsam unter Einhaltung eines pH-Wertes von 11 bis 11,5 mittels Natronlauge und einer Temperatur von höchsten 60° C in eine Lösung von 780 Teilen Thioglykol und 400 Teilen Natriumhydroxid in 1500 Teilen Wasser. Man rührt noch einige Zeit bei etwa 50° C nach und destilliert sodann das Wasser unter reduziertem Druck ab, digeriert den Rückstand in 1500 Volumenteilen Methanol, filtriert von Ungelöstem ab und isoliert nach Entfernen des Lösemittels aus dem Rückstand das γ-(β′-Hydroxyethyl-thio)-propylamin durch fraktionierte Destillation bei 132-140° C/3 mbar.

270 Teile dieser Verbindung werden in 243 Teilen einer 30%igen Salzsäure gelöst. Man gibt 2 Teile Natriumwolframat hinzu und anschließend langsam 395 Teile 35%iges wäßriges Wasserstoffperoxid, wobei die stark exotherme Reaktion unterhalb 98° C gehalten wird.

Nach dem Abkühlen wird der erhaltene Ansatz des Hydrochlorids des γ-(β′-Hydroxyethylsulfonyl)-propylamins mit 200 Teilen Wasser versetzt. Man stellt mit Natronlauge einen pH-Wert von 9 ein und gibt eine Lösung von 236 Teilen 5-Nitrobenzol-1,3-di-(carbonsäurechlorid) mit 240 Volumenteilen Aceton unter Einhaltung eines pH-Wertes von 7 bis 9 mittels Natronlauge und einer Temperatur von etwa 30° C hinzu. Nach Beendigung der Reaktion kühlt man den Ansatz auf 0 bis 5° C ab und isoliert das gebildete 5-Nitrobenzol-1,3-di-{carbonsäure-[γ-(β′-hydroxyethylsulfonyl)-propylamid]} durch Filtration; es besitzt einen Schmelzpunkt von 101-104° C.

Die erhaltene Nitrobenzol-dicarbonsäureamid-Verbindung wird in 1200 Teilen Wasser suspendiert und katalytisch mit Wasserstoff und Raney-Nickel bei 25 bis 60° C und einem Wasserstoffüberdruck von etwa 40 bar zur Aminoverbindung der Formel (19A) reduziert. Nach beendeter Reduktion wird der Katalysator abfiltriert. Die Verbindung (19A) kristallisiert beim Abkühlen des Filtrats auf 0 bis 5° C aus. Es besitzt einen Schmelzpunkt von 145 bis 147° C und ist dünnschichtchromatographisch einheitlich.

**Beispiel B**

Das unter Beispiel A hergestellte 5-Aminobenzol-1,3-bis-[γ-(β′-hydroxyethylsulfonyl)-N-propyl]-carbonsäureamid kann in dessen faserreaktiven Derivate, wie beispielsweise deren Bis-sulfato-, -phosphato- oder -acetyloxy-Verbindung, auf übliche Weise übergeführt werden, so beispielsweise durch Umsetzung in 100%iger Schwefelsäure oder 100%iger Phosphorsäure oder in Eisessig.
Sowohl die β-Hydroxyethylsulfonyl- als auch die β-Sulfato-, β-Phosphato- und β-Acetyloxyethylsulfonyl-Verbindungen dieses 5-Nitro-1,3-dicarbonsäureamids dienen als Diazokomponente für die Synthese der erfindungsgemäßen Farbstoffe. Wird die β-Hydroxyethylsulfonyl-Verbindung gemäß Formel (19A) als Diazokomponente in die Synthese der erfindungsgemäßen Farbstoffe eingesetzt, so lassen sich analog zu bekannten Verfahrensweisen die β-Hydroxyethylsulfonyl-Gruppen entweder in Zwischenverbindungen während der Synthese des Azofarbstoffes oder im fertiggestellten Azofarbstoff selbst in die entsprechenden β-Sulfato-, β-Phosphato- oder β-Acetyloxy-Derivate überführen.

**Beispiel 1**

47,9 Teile der Verbindung der Formel (19A) von Beispiel A werden in 150 Teile 100%iger Schwefelsäure eingetragen. Man gibt 25 Teile 65%iges Oleum hinzu; hierbei erwärmt sich die Mischung auf 70° C und rührt noch 3 Stunden bei 70 bis 75° C nach. Sodann gießt man die erhaltene Lösung auf ein Gemisch von 1200 Teilen Wasser und 800 Teilen Eis und neutralisiert einen Teil der Schwefelsäure mittels 100 Teilen Calciumcarbonat. Die Anilinverbindung wird sodann in üblicher Weise mittels 21 Volumenteilen einer 5n-Natriumnitritlösung diazotiert. Zu der Diazoniumsalzlösung gibt man 42,3 Teile 1-Benzoylamino-8-naphthol-3,6-disulfonsäure und führt die Kupplungsreaktion bei einem pH-Wert von 5,5 bis 6 durch. Man rührt eine Stunde nach, filtriert vom Calciumsulfat ab und dampft das Filtrat unter reduziertem Druck ein.

Man erhält das erfindungsgemäße Natriumsalz der Verbindung der Formel

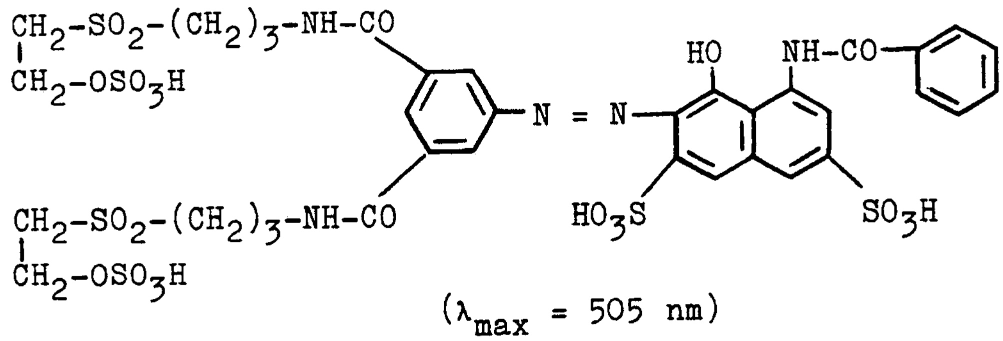

($\lambda_{max}$ = 505 nm)

als salzhaltiges rotes Pulver. Die erfindungsgemäße Azoverbindung zeigt sehr gute faserreaktive Farbstoffeigenschaften und färbt nach den für faserreaktive Farbstoffe üblichen Applikations- und Fixierverfahren die in der Beschreibung genannten Materialien, insbesondere Baumwolle und Zellwolle, in farbtiefen roten Tönen mit guten Echtheitseigenschaften, von denen insbesondere die guten Naßechtheiten, beispielsweise die guten Schweißechtheiten, hervorgehoben werden können.

**Beispiel 2**

Man löst die erfindungsgemäße Azoverbindung des Beispieles 1 in Wasser und stellt mittels Natronlauge einen pH-Wert von 10,5 ein, hält ihn noch etwa 15 Minuten durch Zugabe von Natronlauge. Sodann stellt man die Lösung mit wäßriger Salzsäure auf einen pH-Wert von 5 und salzt die hergestellte erfindungsgemäße Vinylsulfonyl-Verbindung mittels Natriumchlorid aus.

Die als Natriumsalz isolierte erfindungsgemäße Verbindung der Formel

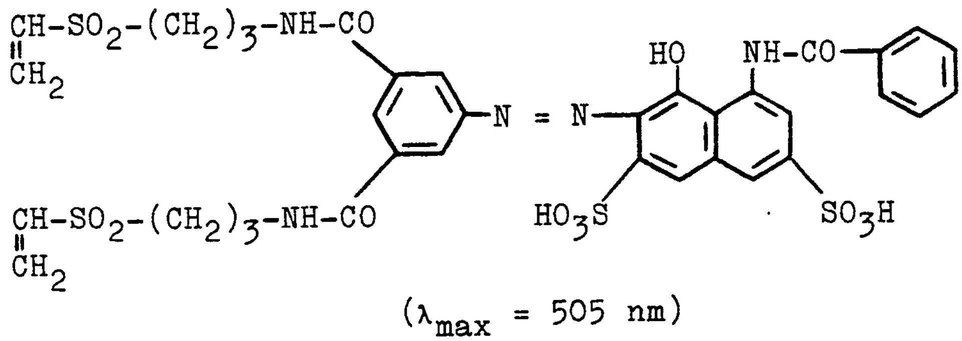

($\lambda_{max}$ = 505 nm)

zeigt ebenfalls sehr gute Farbstoffeigenschaften und färbt beispielsweise Cellulosefasermaterialien nach den für faserreaktive Farbstoffe üblichen Applikations- und Fixiermethoden in kräftigen roten Tönen mit den im Beispiel 1 angegebenen guten Echtheitseigenschaften.

**Beispiel 3**

8,82 Teile der erfindungsgemäßen Azoverbindung des Beispieles 2 werden in 150 Teilen Wasser bei 70° C gelöst. Man stellt einen pH-Wert von 6,5 ein, gibt 3,2 Teile Natriumthiosulfat hinzu und rührt den Ansatz noch 3 Stunden bei 70° C unter Einhaltung eines pH-Wertes von 6,5 mittels verdünnter wäßriger Essigsäure. Die erfindungsgemäße Thiosulfatoverbindung der Formel

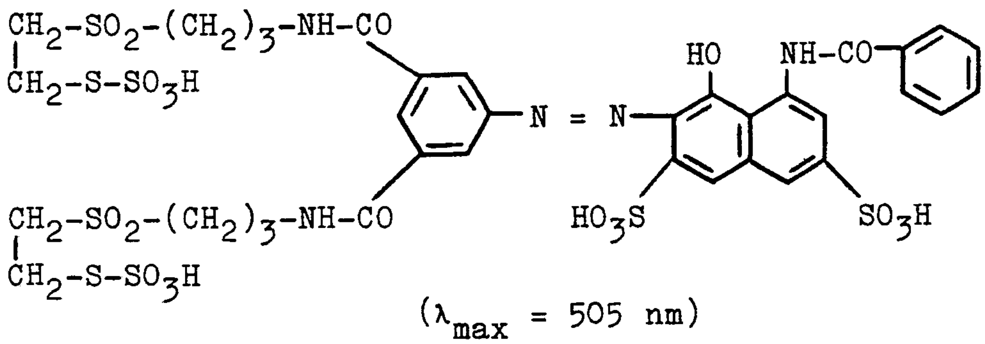

($\lambda_{max}$ = 505 nm)

wird in Form ihres Natriumsalzes durch Eindampfen der Lösung (beispielsweise Sprühtrocknung) oder durch Aussalzen mittels einem Elektrolytsalz isoliert. Sie besitzt ebenfalls gute faserreaktive Farbstoffeigenschaften und liefert farbtiefe rote Färbungen mit den guten Echtheiten, die im Beispiel 1 genannt sind.

**Beispiel 4**

Man trägt 47,9 Teile der Ausgangsverbindung der Formel (19A) von Beispiel A in 400 Teile Eisessig ein, setzt 1 Teil 96,5%iger Schwefelsäure hinzu und rührt den Ansatz zwei Stunden bei 60 bis 70° C. Überschüssige Essigsäure und Wasser werden unter reduziertem Druck abdestilliert. Die verbleibende Schmelze wird auf ein Gemisch aus Eis und Wasser gegeben und mit 15 Teilen 96,5 %iger Schwefelsäure versetzt. Man diazotiert die Anilinverbindung in üblicher Weise mittels Natriumnitrit, gibt sodann zu der

Diazoniumsalzsuspension 36,1 Teile 1-Acetylamino-8-naphthol-3,6-disulfonsäure hinzu, stellt den Ansatz auf einen pH-Wert von 5,5, rührt eine Stunde nach und dampft die Syntheselösung sodann unter reduziertem Druck ein.

Man erhält die erfindungsgemäße Azoverbindung der Formel

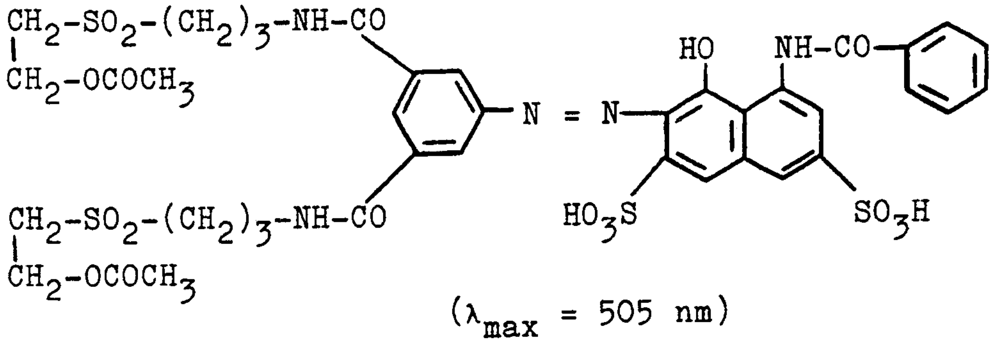

($\lambda_{max}$ = 505 nm)

als Alkalimetallsalz (Natriumsalz). Sie besitzt ebenfalls gute Farbstoffeigenschaften und färbt nach den Applikations-und Fixiermethoden für faserreaktive Farbstoffe die in der Beschreibung genannten Materialien, insbesondere Cellulosefasermaterialien, in kräftigen roten Tönen mit den im Beispiel 1 angegebenen guten Echtheitseigenschaften.

**Beispiele 5 bis 34**

In den nachfolgenden Tabellenbeispielen sind weitere erfindungsgemäße Azoverbindungen mit Hilfe der Formelreste der allgemeinen Formel (A)

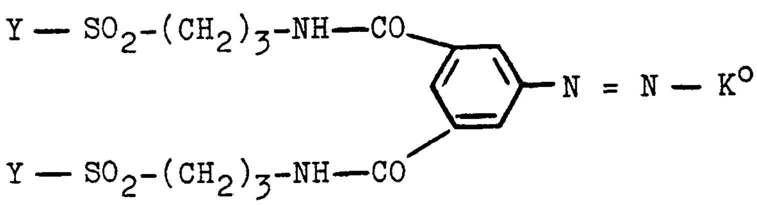

(A)

beschrieben. Sie lassen sich in erfindungsgemäßer Weise, beispielsweise analog den obigen Ausführungsbeispielen durch Kupplungsreaktion der entsprechenden, aus dem jeweiligen Tabellenbeispiel ersichtlichen Diazokomponente und Kupplungskomponente herstellen. Sie besitzen sehr gute Farbstoffeigenschaften und liefern auf den in der Beschreibung genannten Materialien, insbesondere Cellulosefasermaterialien, nach den in der Technik für faserreaktive Farbstoffe üblichen Applikations- und Fixierverfahren farbstarke Färbungen und Drucke in dem für das jeweilige Tabellenbeispiel für die Färbung auf Baumwolle angegebenen Farbton.

| Bsp. | Rest Y | Rest $K^o$ | Farbton |
|---|---|---|---|
| 5 | ß-Sulfato-ethyl | 4,6-Disulfo-1-benzoyl-amino-8-hydroxy-naphth-7-yl | rot (497) |
| 6 | Vinyl | dito | rot (497) |
| 7 | Vinyl | 3,6-Disulfo-1-propionyl-amino-8-hydroxy-naphth-7-yl | rot (503) |
| 8 | Sulfatoethyl | dito | rot (503) |
| 9 | ß-Sulfato-ethyl | 3,6-Disulfo-1-amino-2-(2',5'-disulfo-phenylazo)-8-hydroxy-naphth-7-yl | marineblau (598) |
| 10 | Vinyl | 3,6-Disulfo-1-amino-2-(2',4'-disulfo-phenylazo)-8-hydroxy-naphth-7-yl | marineblau (600) |
| 11 | ß-Sulfato-ethyl | 3,6-Disulfo-1-amino-2-(4'-nitrophenylazo)-8-hydroxy-naphth-7-yl | schwarz (604) |
| 12 | dito | 3,6-Disulfo-1-amino-2-(4'-ß-sulfatoethylsulfonyl)-phenylazo-8-hydroxy-naphth-7-yl | marineblau (598) |
| 13 | dito | 5-Sulfo-1-hydroxy-naphth-2-yl | gelbstichig rot (494) |
| 14 | dito | 4-Sulfo-1-amino-naphth-2-yl | orange (466) |
| 15 | dito | 6-Carboxy-2-hydroxy-naphth-1-yl | goldgelb (478) |
| 16 | dito | 4-Sulfo-1-hydroxy-naphth-2-yl | orange (485) |
| 17 | dito | 3-Sulfo-7-acetylamino-1-hydroxy-naphth-2-yl | rotstichig orange (490) |
| 18 | dito | 3-Sulfo-7-benzoylamino-1-hydroxy-naphth-2-yl | rotstichig orange (491) |
| 19 | dito | 3-Sulfo-6-acetylamino-1-hydroxy-naphth-2-yl | orange (477) |
| 20 | dito | 3-Sulfo-6-benzoylamino-1-hydroxy-naphth-2-yl | orange (479) |
| 21 | dito | 1-(4'-Sulfophenyl)-3-methyl-pyrazol-5-on-4-yl | gelb (384) |
| 22 | Vinyl | 1-(4'-Sulfophenyl)-3-carboxy-pyrazol-5-on-4-yl | gelborange (419) |
| 23 | ß-Phosphato-ethyl | 1-(4'-ß-Sulfatoethylsulfonyl)-3-methyl-pyrazol-5-on-4-yl | gelb (384) |

| Bsp. | Rest Y | Rest $K^o$ | Farbton |
|---|---|---|---|
| 24 | ß-Acetyl-oxyethyl | 3,6-Disulfo-1-amino-2-(2'-sulfo-5'-ß-sulfatoethyl-sulfonyl-phenylazo)-8-hydroxy-naphth-7-yl | marineblau (602) |
| 25 | dito | 3,6-Disulfo-1-amino-2-(2'-sulfo-4'-ß-sulfatoethyl-sulfonyl-phenylazo)-8-hydroxy-naphth-7-yl | marineblau (604) |
| 26 | ß-Sulfato-ethyl | 3-Sulfo-7-(2'-fluor-4'-diethylamino-1',3',5'-triazin-6'-yl)-amino-1-hydroxy-naphth-2-yl | scharlach (491) |
| 27 | | 3-Sulfo-7-(2'-chlor-4'-morpholino-1',3',5'-triazin-6'-yl)-amino-1-hydroxy-naphth-2-yl | scharlach (491) |
| 28 | Vinyl | 3,6-Disulfo-1-(2'-chlor-4'-methoxy-1',3',5'-triazin-6'-yl)-amino-8-hydroxy-naphth-7-yl | rot (506) |
| 29 | Vinyl | 3,6-Disulfo-1-[2'-fluor-4'-(3"-sulfophenyl)-amino-1',3',5'-triazin-6'-yl]-amino-8-hydroxy-naphth-7-yl | rot (506) |
| 30 | Vinyl | 3,6-Disulfo-1-[2'-chlor-4'-(4"-ß-sulfatoethylsulfonyl-phenyl)-amino-1',3',5'-triazin-6'-yl]-amino-8-hydroxy-naphth-7-yl | rot (507) |
| 31 | ß-Sulfato-ethyl | 3-Sulfo-6-(2',4'-dichlor-1',3',5'-triazin-6'-yl)-amino-1-hydroxy-naphth-2-yl | orange (480) |
| 32 | dito | 3-Sulfo-6-(2'-fluor-4'-morpholino-1',3',5'-triazin-6'-yl)-amino-1-hydroxy-naphth-2-yl | orange (480) |
| 33 | dito | N-(ß-Sulfoethyl)-4-methyl-2-hydroxy-pyrid-6-on-3-yl | gelb (405) |
| 34 | dito | 3-Sulfo-7-[2'-fluor-4'-(3"-ß-sulfatoethylsulfonyl-phenyl)-amino-1',3',5'-triazin-6'-yl]-amino-1-hydroxy-naphth-2-yl | scharlach (491) |

**Beispiel 35**

Man stellt gemäß den Angaben des Beispieles 1 das Diazoniumsalz des Sulfatoderivates der Ausgangsverbindung der Formel (19A) her und filtriert anschließend von Calciumsulfat ab. Das Filtrat wird durch

Zugabe von Eis bei einer Temperatur von 5 °C mittels Natriumbicarbonat auf einen pH-Wert von 1,8 gestellt und anschließend mit 29 Teilen 1-Amino-8-naphthol-3,6-disulfonsäure versetzt. Man rührt 4 Stunden nach und gibt anschließend bei etwa 10 °C eine auf üblichem Wege hergestellte Diazoniumsalzlösung von 29,5 Teilen 2-Methoxy-5-(β-sulfatoethylsulfonyl)-anilin hinzu. Man stellt den pH-Wert auf 6 und rührt bis zur Beendigung der Kupplungsreaktion unter Einhaltung dieses pH-Wertes nach.

Die erhaltene Syntheselösung wird eingedampft oder sprühgetrocknet. Man erhält ein schwarzes, elektrolythaltiges Pulver der erfindungsgemäßen Alkalimetallverbindung (Natriumverbindung) der Formel

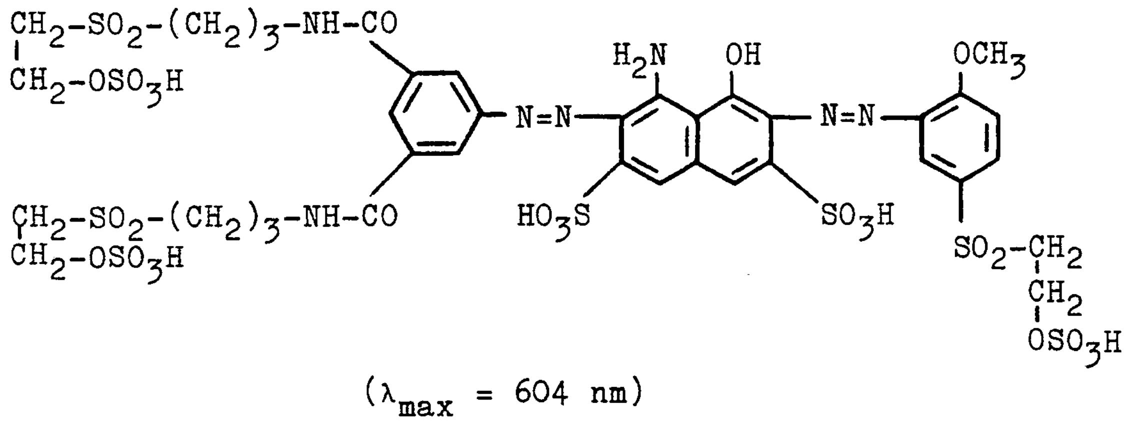

($\lambda_{max}$ = 604 nm)

die gute Farbstoffeigenschaften besitzt und nach den in der Technik für faserreaktive Farbstoffe üblichen Anwendungsmethoden die in der Beschreibung genannten Materialien, insbesondere Cellulosefasermaterialien, in grünstichig-marineblauen Tönen mit hoher Farbstärke und guten Echtheiten färbt.

**Beispiele 36 bis 54**

In den nachfolgenden Tabellenbeispielen sind weitere erfindungsgemäße Disazoverbindungen mit Hilfe der Formelreste der allgemeinen Formel (B)

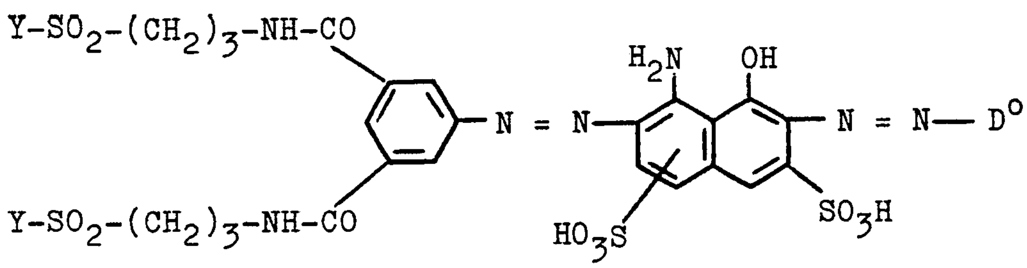

(B)

beschrieben. Sie lassen sich in erfindungsgemäßer Weise, beispielsweise analog dem obigen Ausführungsbeispiel 35, aus den Ausgangsverbindungen, die sich von den in dem entsprechenden Tabellenbeispiel angegebenen Komponenten ersichtlich sind, herstellen. Sie besitzen sehr gute Farbstoffeigenschaften und liefern auf den in der Beschreibung genannten Materialien, insbesondere Cellulosefasermaterialien, nach den für faserreaktive Farbstoffe üblichen Anwendungsverfahren farbstarke Färbungen und Drucke in dem für das jeweilige Tabellenbeispiel für die Färbung auf Baumwolle angegebenen Farbton.

| Bsp. | Rest Y | Rest D° | Stellung der der Gruppe $-SO_3H$ in ... | Farbton |
|---|---|---|---|---|
| 36 | ß-Sulfato-ethyl | 3-(ß-Sulfatoethyl-sulfonyl)-phenyl | 3- | marineblau (589) |
| 37 | Vinyl | 2-Sulfo-4-(ß-sulf-fatoethylsulfonyl)-phenyl | 3- | marineblau (587) |
| 38 | ß-Sulfato-ethyl-sulfonyl | 2-Sulfo-5-(2'-fluor-4'-methoxy-1',3',5'-triazin-6'-yl)-amino-phenyl | 4- | marineblau (574) |
| 39 | dito | dito | 3- | marineblau (591) |
| 40 | dito | 2-Sulfo-5-(2'-chlor-4'-methoxy-1',3',5'-triazin-6'-yl)-amino-phenyl | 3- | marineblau (590) |

| Bsp. | Rest Y | Rest $D^0$ | Stellung der der Gruppe $-SO_3H$ in ... | Farbton |
|---|---|---|---|---|
| 41 | dito | dito | 4- | marineblau (576) |
| 42 | Vinyl | 2-Sulfo-5-[2'-chlor-4'-N,N-di-(ß-hydroxy-ethyl)-amino-1',3',5'-triazin-6'-yl]-amino-phenyl | 3- | marineblau (593) |
| 43 | ß-Sulfato-ethyl-sulfonyl | 2-Sulfo-5-[2'-fluor-4'-(3"-ß-sulfatoethyl-sulfonyl-phenyl)-amino-1',3',5'-triazin-6'-yl]-amino-phenyl | 3- | marineblau (591) |
| 44 | dito | 2-Sulfo-5-(2'-fluor-4'-morpholino-1',3',5'-triazin-6'-yl)-amino-phenyl | 3- | marineblau (593) |
| 45 | dito | 2-Sulfo-5-[2'-fluor-4'-(3"-ß-sulfoethyl-sulfonyl-phenyl)-amino-1',3',5'-triazin-6'-yl]-amino-phenyl | 3- | marineblau (591) |
| 46 | dito | 1-Sulfo-6-(ß-sulfato-ethylsulfonyl)-naphth-2-yl | 3- | marineblau (619) |
| 47 | dito | 4-Sulfo-phenyl | 3- | marineblau (594) |
| 48 | dito | 2-Sulfo-phenyl | 3- | marinablau (595) |
| 49 | dito | 1,5-Disulfo-naphth-2-yl | 3- | marineblau (616) |
| 50 | dito | 4,8-Disulfo-naphth-2-yl | 3- | marineblau (619) |
| 51 | dito | 2-Carboxy-phenyl | 3- | marineblau (597) |
| 52 | dito | 4-Carboxy-phenyl | 3- | marineblau (597) |
| 53 | dito | 4-(ß-Sulfatoethyl-sulfonyl)-phenyl | 3- | marineblau (591) |
| 54 | dito | 2,5-Dimethoxy-4-(ß-sulfatoethyl-sulfonyl)-phenyl | 3- | marineblau (612) |

**Ansprüche**

1. Eine Verbindung entsprechend der allgemeinen Formel (1)

D - N = N - K° (1)

in welcher bedeuten:

D ist ein Rest der allgemeinen Formel (2)

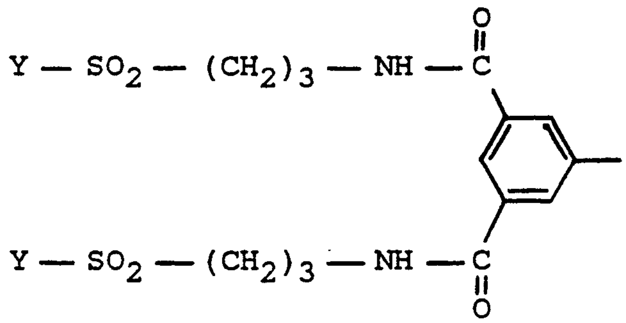

(2)

in welcher

Y die Vinylgruppe oder die β-Sulfatoethyl-, β-Acetyloxyethyl-, β-Thiosulfatoethyl oder β-Phosphatoethyl-Gruppe ist;

K° ist der Rest einer Kupplungskomponente der Benzol- oder Naphthalinreihe oder der heterocyclischen Reihe oder der Acetoacetarylid-Reihe oder

K° ist ein Rest der allgemeinen Formel (3)

- K - G - Q (3)

in welcher

K ein bivalenter Rest aus der Benzol- oder Naphthalin-Reihe ist,

G für eine Gruppe der Formel

- N - CO -
  R

oder - N - $SO_2$ -
       R

steht, in welchen

R ein Wasserstoffatom oder eine Alkylgruppe von 1 bis 4 C-Atomen ist, oder

G eine Azogruppe der Formel -N = N- ist und

Q einen aliphatischen oder aromatischen Rest bedeutet, der seinerseits farbstoffübliche Reste enthält, oder

K° ist ein Rest der allgemeinen Formel -K*-Z ,

in welcher

K* der bivalente Rest einer Kupplungskomponente der Benzol- oder Naphthalin-Reihe oder der heterocyclischen Reihe oder der Acetoacetarylid-Reihe ist und

Z ein faserreaktiver Acylaminorest, bevorzugt ein Rest der allgemeinen Formel (4)

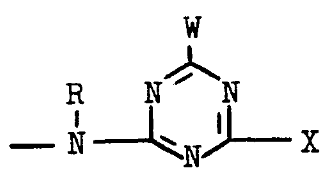

(4)

ist, in welcher

R eine der obengenannten Bedeutungen hat,

X für ein Fluor- oder Chloratom oder den Morpholinorest oder eine Gruppe der allgemeinen Formel $-O-R^{\alpha}$ oder eine Aminogruppe der allgemeinen Formel $-NR^{\alpha}R^{\beta}$ steht,

in welchen

$R^{\alpha}$ ein Wasserstoffatom oder ein aliphatischer oder aromatischer Rest ist, der farbstoffübliche Reste enthält, und

$R^{\beta}$ ein Wasserstoffatom oder eine Alkylgruppe von 1 bis 4 C-Atomen ist, die durch Hydroxy, Sulfato, Sulfo, Carboxy, Phosphato, Phenyl, Methoxy oder Ethoxy substituiert sein kann, und

W einen der für X angegebenen Bedeutungen besitzt, wobei X und W zueinander gleich oder voneinander

verschieden sein können.

2. Verbindung nach Anspruch 1, dadurch gekennzeichnet, daß in der Gruppe der Formel (4) der Rest X eine Aminogruppe $-NR^{\alpha}R^{\beta}$ ist, in welcher $R^{\alpha}$ ein Wasserstoffatom oder eine Alkylgruppe von 1 bis 4 C-Atomen ist, die durch Hydroxy, Sulfato, Sulfo, Carboxy, Phosphato, Phenyl, Methoxy oder Ethoxy substituiert sein kann, oder ein Phenylrest ist, der durch Substituenten substituiert sein kann, die aus der Gruppe Alkyl von 1 bis 4 C-Atomen, Alkoxy von 1 bis 4 C-Atomen, Carboxy, Sulfo, einer Gruppe der Formel $-SO_2-Y$ mit Y einer der obengenannten Bedeutungen, Chlor und Brom ausgewählt sind, und $R^{\beta}$ die in Anspruch 1 genannte Bedeutung hat.

3. Verbindung nach Anspruch 1 oder 2 entsprechend der allgemeinen Formel (5a)

$$D-N=N\{E-N=N\}_{v}\!-K^{o} \qquad (5a)$$

in welcher
D und K° die in Anspruch 1 genannten Bedeutungen haben,
E den bivalenten Rest einer kupplungsfähigen und diazotierbaren Verbindung bedeutet und
v für die Zahl Null oder 1 steht.

4. Verbindung nach Anspruch 1 entsprechend der allgemeinen Formel (5b)

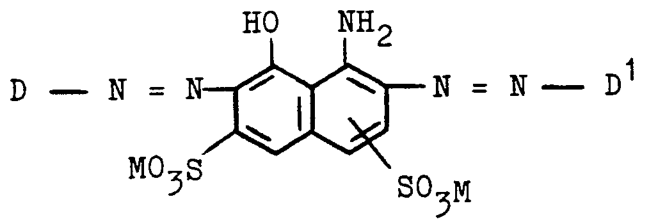

(5b)

in welcher
M für ein Wasserstoffatom oder ein Alkalimetall steht,
D die in Anspruch 1 genannte Bedeutung besitzt und
$D^1$ ein Rest einer Diazokomponente der Benzol- oder Naphthalinreihe ist, der durch in faserreaktiven Farbstoffen übliche Substituenten substituiert sein kann, beispielsweise durch Substituenten, die aus der folgenden Gruppe vom Substituenten ausgewählt sind: Alkyl von 1 bis 4 C-Atomen, wie Methyl und Ethyl, Alkoxy von 1 bis 4 C-Atomen wie Methoxy und Ethoxy, Carboxy, Sulfo, Halogen, wie Chlor und Brom, Trifluormethyl, Nitro, Benzoylamino, eine Gruppe der oben definierten allgemeinen Formel $-SO_2-Y$ , eine faserreaktive aliphatische oder heterocyclische Acylaminogruppe Z, beispielsweise eine Gruppe der oben genannten und definierten allgemeinen Formel (4), Alkanoylamino von 2 bis 5 C-Atomen und Hydroxy, oder $D^1$ ein Rest der allgemeinen Formel (2) ist.

5. Verbindung nach Anspruch 1 entsprechend der allgemeinen Formel (5c)

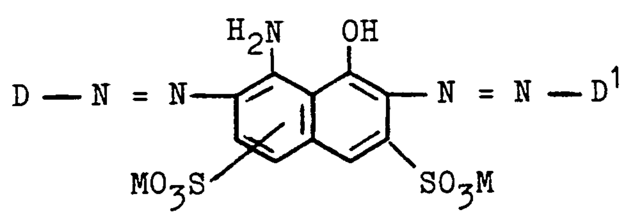

(5c)

in welcher
M für ein Wasserstoffatom oder ein Alkalimetall steht,
D die in Anspruch 1 genannte Bedeutung besitzt und
$D^1$ ein Rest einer Diazokomponente der Benzol- oder Naphthalinreihe ist, der durch in faserreaktiven Farbstoffen übliche Substituenten substituiert sein kann, beispielsweise durch Substituenten, die aus der folgenden Gruppe vom Substituenten ausgewählt sind: Alkyl von 1 bis 4 C-Atomen, wie Methyl und Ethyl, Alkoxy von 1 bis 4 C-Atomen wie Methoxy und Ethoxy, Carboxy, Sulfo, Halogen, wie Chlor und Brom, Trifluormethyl, Nitro, Benzoylamino, eine Gruppe der oben definierten allgemeinen Formel $-SO_2-Y$ , eine faserreaktive aliphatische oder heterocyclische Acylaminogruppe Z, beispielsweise eine Gruppe der oben genannten und definierten allgemeinen Formel (4), Alkanoylamino von 2 bis 5 C-Atomen und Hydroxy, oder $D^1$ ein Rest der allgemeinen Formel (2) ist.

6. Verbindung nach mindestens einem der Ansprüche 1 bis 3, dadurch gekennzeichnet, daß W ein

Fluor- oder Chloratom bedeutet.

7. Verfahren zur Herstellung einer in Anspruch 1 genannten und definierten Verbindung der allgemeinen Formel (1), dadurch gekennzeichnet, daß man ein Diazoniumsalz eines Amins der allgemeinen Formel (15)

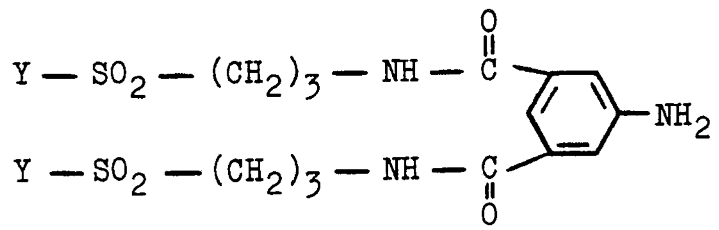

(15)

in welcher Y die in Anspruch 1 genannten Bedeutungen haben, mit einer Kupplungskomponente der allgemeinen Formel H-K° mit K° der in Anspruch 1 genannten Bedeutung kuppelt,
oder daß man eine Verbindung der allgemeinen Formel (17)

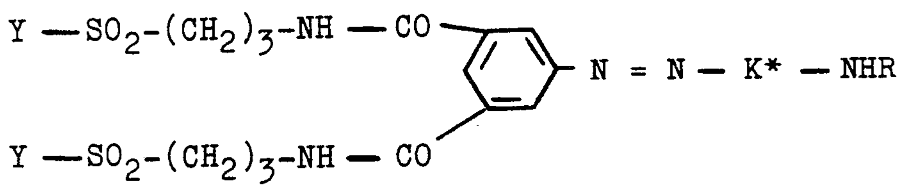

(17)

mit Y, K* und R der in Anspruch 1 genannten Bedeutung mit einer Verbindung der allgemeinen Formel Hal-$Z^1$ mit Hal und $Z^1$ der obengenannten Bedeutung umsetzt.

8. Verfahren nach Anspruch 7, dadurch gekennzeichnet, daß man eine Verbindung der allgemeinen Formel (18)

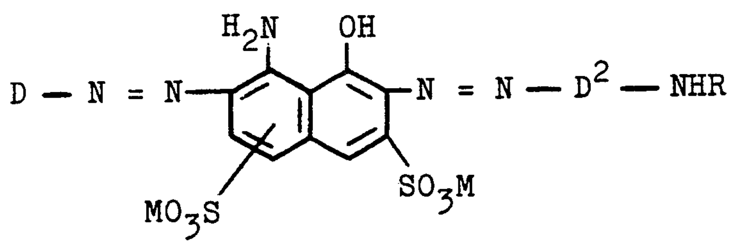

(18)

in welcher D, R und M die in Anspruch 1 und 5 genannten Bedeutungen haben und
$D^2$ ein Phenylen- oder Naphthylenrest ist, der durch in faserreaktiven Farbstoffen übliche Substituenten substituiert sein kann, beispielsweise durch Substituenten aus der Gruppe Alkyl von 1 bis 4 C-Atomen, Alkoxy von 1 bis 4 C-Atomen, Carboxy, Sulfo, Halogen, Trifluormethyl, Nitro, Benzoylamino, eine Gruppe der oben definierten allgemeinen Formel $-SO_2-Y$ , Alkanoylamino von 2 bis 5 C-Atomen und Hydroxy,
mit einer Verbindung der allgemeinen Formel Hal-$Z^1$ mit Hal und $Z^1$ der obengenannten Bedeutung umsetzt.

9. Verfahren nach Anspruch 6 zur Herstellung einer Verbindung der in Anspruch 1 genannten und definierten allgemeinen Formel (1), in welcher jedoch beide Y β-Sulfatoethyl-Gruppen bedeuten und/oder eine der möglichen Substituenten in K° einen β-Sulfatoethyl-Rest enthält, dadurch gekennzeichnet, daß man eine Verbindung entsprechend der allgemeinen Formel (1) der in Anspruch 1 angegebenen Bedeutung, in welcher jedoch beide Y β-Hydroxyethyl-Gruppen sind und/oder einer der für K° angegebenen Substituenten ein β-Hydroxyethyl-Rest enthält, mit einem Sulfatierungsmittel verestert.

10. Verfahren zur Herstellung einer in Anspruch 4 definierten Disazoverbindung der allgemeinen Formel (5c), dadurch gekennzeichnet, daß man ein Diazoniumsalz eines Amins der allgemeinen Formel $D^1-NH_2$ mit $D^1$ der in Anspruch 4 angegebenen Bedeutung mit einer Verbindung der allgemeinen Formel (16)

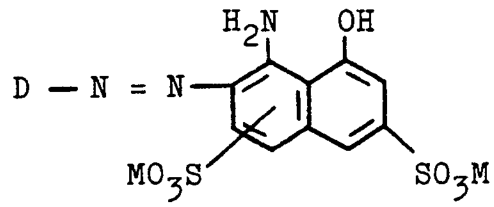

(16)

in welcher D und M die in Anspruch 4 genannten Bedeutungen haben, kuppelt und im Falle, daß in den Ausgangsverbindungen der Formeln $D^1$-$NH_2$ und (16) die Reste D und $D^1$ eine oder mehrere β-Hydroxyethylsulfonyl-Gruppen enthalten, in der synthetisierten Disazoverbindung entsprechend der allgemeinen Formel (5c), in welcher jedoch eine oder mehrere β-Hydroxyethylsulfonyl-Gruppen enthalten sind, diese β-Hydroxyethylsulfonyl-Gruppen mittels einem Sulfatierungsmittel in die β-Sulfatoethylsulfonyl-Gruppen überführt.

11. Verwendung eines Farbstoffes von Anspruch 1 zum Färben und Bedrucken von hydroxy- und/oder carbonamidgruppenhaltigem Material, insbesondere Fasermaterial.

12. Verfahren zum Färben und Bedrucken von hydroxy- und/oder carbonamidgruppenhaltigem Material, insbesondere Fasermaterial, bei welchem man einen Farbstoff auf das Material aufbringt und ihn mittels Wärme oder mit Hilfe eines alkalisch wirkenden Mittels oder mittels Wärme und mit Hilfe eines alkalischen Mittels fixiert, dadurch gekennzeichnet, daß man als Farbstoff eine Verbindung entsprechend der allgemeinen Formel (1) von Anspruch 1 verwendet.

13. Eine Verbindung entsprechend der allgemeinen Formel (19)

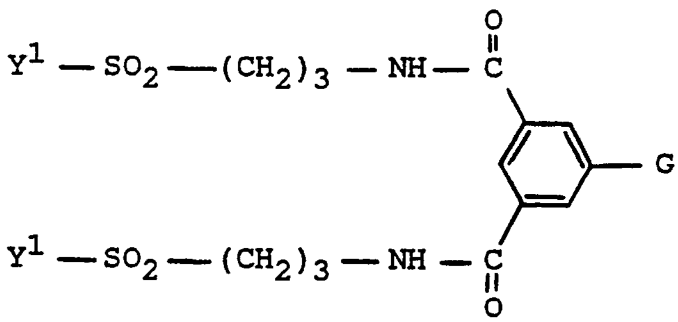

(19)

in welcher $Y^1$ die Vinylgruppe oder die β-Sulfatoethyl-, β-Acetyloxyethyl-, β-Thiosulfatoethyl-, β-Phosphatoethyl-oder β-Hydroxyethyl-Gruppe ist und G für die Nitrogruppe oder die Aminogruppe steht.

14. Verfahren zur Herstellung einer in Anspruch 13 genannten und definierten Verbindung der allgemeinen Formel (19), dadurch gekennzeichnet, daß man γ-(β′-Hydroxyethylsulfonyl)-propylamin in zweifach molarer Menge mit 5-Nitro-benzol-1,3-di-(carbonsäurechlorid) umsetzt und im Falle der Herstellung einer Verbindung (19) mit G der Aminogruppe die Nitrogruppe reduziert und ggfs. zur Synthese einer Verbindung der allgemeinen Formel (19) mit $Y^1$ einer anderen der angegebenen Bedeutung als β-Hydroxyethyl die β-Hydroxyethyl-Gruppen analog bekannten Verfahrensweisen in andere solche Gruppen überführt.

15. Verwendung einer Verbindung der allgemeinen Formel (19) von Anspruch 14 zur Synthese von faserreaktiven Farbstoffen, insbesondere Azofarbstoffen.

Geänderte Patentansprüche gemäss Regel 86(2) EPÜ

$D^1$ ein Rest einer Diazokomponente der Benzol- oder Naphthalinreihe ist, der durch in faserreaktiven Farbstoffen übliche Substituenten substituiert sein kann, beispielsweise durch Substituenten, die aus der folgenden Gruppe vom Substituenten ausgewählt sind: Alkyl von 1 bis 4 C-Atomen, Alkoxy von 1 bis 4 C-Atomen, Carboxy, Sulfo, Halogen, wie Chlor und Brom, Trifluormethyl, Nitro, Benzoylamino, Alkanoylamino von 2 bis 5 C-Atomen, Hydroxy, eine Gruppe der allgemeinen Formel -$SO_2$-Y und eine faserreaktive aliphatische oder heterocyclische Acylaminogruppe Z, beispielsweise eine Gruppe der allgemeinen Formel (4), oder
$D^1$ ein Rest der allgemeinen Formel (2) ist.

5. Verbindung nach Anspruch 1 entsprechend der allgemeinen Formel (5c)

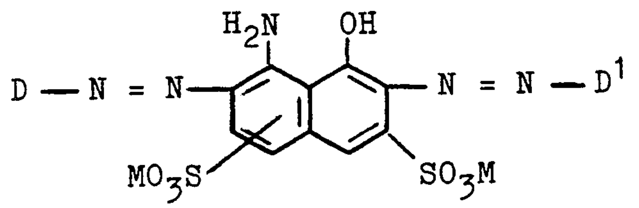

(5c)

in welcher
M für ein Wasserstoffatom oder ein Alkalimetall steht,
D die in Anspruch 1 genannte Bedeutung besitzt und
$D^1$ ein Rest einer Diazokomponente der Benzol- oder Naphthalinreihe ist, der durch in faserreaktiven Farbstoffen übliche Substituenten substituiert sein kann, beispielsweise durch Substituenten, die aus der folgenden Gruppe von Substituenten ausgewählt sind: Alkyl von 1 bis 4 C-Atomen, Alkoxy von 1 bis 4 C-Atomen, Carboxy, Sulfo, Halogen, wie Chlor und Brom, Trifluormethyl, Nitro, Benzoylamino, Alkanoylamino von 2 bis 5 C-Atomen, Hydroxy, eine Gruppe der allgemeinen Formel $-SO_2-Y$ und eine faserreaktive aliphatische oder heterocyclische Acylaminogruppe Z, beispielsweise eine Gruppe der oben genannten und definierten allgemeinen Formel (4), oder
$D^1$ ein Rest der allgemeinen Formel (2) ist.

6. Verbindung nach mindestens einem der Ansprüche 1 bis 3, dadurch gekennzeichnet, daß W ein Fluor- oder Chloratom bedeutet.

7. Verfahren zur Herstellung einer in Anspruch 1 genannten und definierten Verbindung der allgemeinen Formel (1), dadurch gekennzeichnet, daß man ein Diazoniumsalz eines Amins der allgemeinen Formel (15)

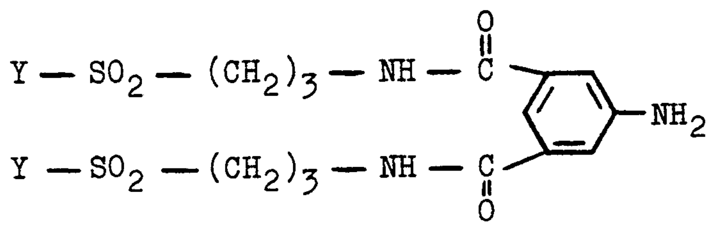

(15)

in welcher Y die in Anspruch 1 genannten Bedeutungen haben, mit einer Kupplungskomponente der allgemeinen Formel H-K° mit K° der in Anspruch 1 genannten Bedeutung kuppelt,
oder daß man eine Verbindung der allgemeinen Formel (17)

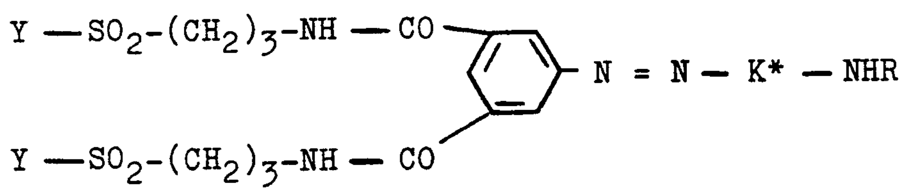

(17)

mit Y, K* und R der in Anspruch 1 genannten Bedeutung mit einer Verbindung der allgemeinen Formel Hal-$Z^1$ mit Hal gleich einem Halogenatom und $Z^1$ gleich einem faserreaktiven Acylrest umsetzt.

8. Verfahren nach Anspruch 7, dadurch gekennzeichnet, daß man eine Verbindung der allgemeinen Formel (18)

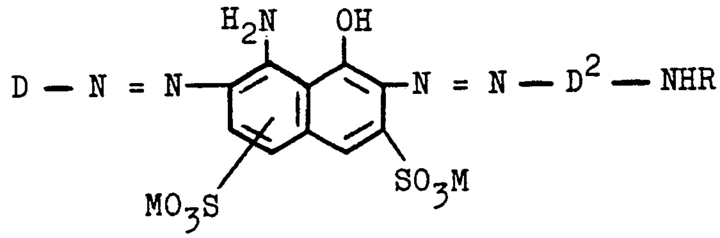

(18)

in welcher D, R und M die in Anspruch 1 und 5 genannten Bedeutungen haben und
$D^2$ ein Phenylen- oder Naphthylenrest ist, der durch in faserreaktiven Farbstoffen übliche Substituenten substituiert sein kann, beispielsweise durch Substituenten aus der Gruppe Alkyl von 1 bis 4 C-Atomen, Alkoxy von 1 bis 4 C-Atomen, Carboxy, Sulfo, Halogen, Trifluormethyl, Nitro, Benzoylamino, Alkanoylamino von 2 bis 5 C-Atomen Hydroxy und eine Gruppe der oben definierten allgemeinen Formel $-SO_2-Y$, mit einer Verbindung der allgemeinen Formel Hal-$Z^1$ mit Hal gleich einem Halogenatom und $Z^1$ gleich einem faserreaktiven Acylrest umsetzt.

9. Verfahren nach Anspruch 7 oder 8 zur Herstellung einer Verbindung der in Anspruch 1 genannten und definierten allgemeinen Formel (1), in welcher jedoch beide Y β-Sulfatoethyl-Gruppen bedeuten und/oder eine der möglichen Substituenten in K° einen β-Sulfatoethyl-Rest enthält, dadurch gekennzeichnet, daß man eine Verbindung entsprechend der allgemeinen Formel (1) der in Anspruch 1 angegebenen Bedeutung, in welcher jedoch beide Y β-Hydroxyethyl-Gruppen sind und/oder einer der für K° angegebenen Substituenten ein β-Hydroxyethyl-Rest enthält, mit einem Sulfatierungsmittel verestert.

10. Verfahren zur Herstellung einer in Anspruch 5 definierten Disazoverbindung der allgemeinen Formel (5c), dadurch gekennzeichnet, daß man ein Diazoniumsalz eines Amins der allgemeinen Formel $D^1-NH_2$ mit $D^1$ der in Anspruch 5 angegebenen Bedeutung mit einer Verbindung der allgemeinen Formel (16)

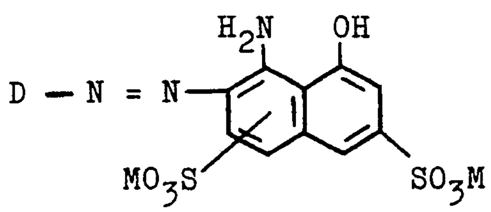

(16)

in welcher D und M die in Anspruch 5 genannten Bedeutungen haben, kuppelt und im Falle, daß in den Ausgangsverbindungen der Formeln $D^1-NH_2$ und (16) die Reste D und $D^1$ eine oder mehrere β-Hydroxyethylsulfonyl-Gruppen enthalten, in der synthetisierten Disazoverbindung entsprechend der allgemeinen Formel (5c), in welcher jedoch eine oder mehrere β-Hydroxyethylsulfonyl-Gruppen enthalten sind, diese β-Hydroxyethylsulfonyl-Gruppen mittels einem Sulfatierungsmittel in die β-Sulfatoethylsulfonyl-Gruppen überführt.

11. Verwendung eines Farbstoffes von Anspruch 1 zum Färben und Bedrucken von hydroxy- und/oder carbonamidgruppenhaltigem Material, insbesondere Fasermaterial.

12. Verfahren zum Färben und Bedrucken von hydroxy- und/oder carbonamidgruppenhaltigem Material, insbesondere Fasermaterial, bei welchem man einen Farbstoff auf das Material aufbringt und ihn mittels Wärme oder mit Hilfe eines alkalisch wirkenden Mittels oder mittels Wärme und mit Hilfe eines alkalischen Mittels fixiert, dadurch gekennzeichnet, daß man als Farbstoff eine Verbindung entsprechend der allgemeinen Formel (1) von Anspruch 1 verwendet.

Europäisches Patentamt

# EUROPÄISCHER RECHERCHENBERICHT

Nummer der Anmeldung

EP 90103080.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | EP - A2 - 0 284 568 (CIBA-GEIGY AG) * Ansprüche 1-14,26-28 * -- | 1-15 | C 09 B 62/507<br>D 06 P 1/384 |
| D,A | EP - A1 - 0 221 013 (CIBA-GEIGY AG) * Ansprüche * ---- | 1-15 | |

RECHERCHIERTE SACHGEBIETE (Int Cl 5)

C 09 B
D 06 P

Der vorliegende Recherchenbericht wurde für alle Patentansprüche erstellt.

| Recherchenort | Abschlußdatum der Recherche | Prüfer |
|---|---|---|
| WIEN | 25-05-1990 | HAUSWIRTH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument